# EUROPEAN PATENT SPECIFICATION

(11) **EP 2 298 282 B1**
(45) Date of publication and mention of the grant of the patent: **15.02.2017**
(21) Application number: 09746712.0
(22) Date of filing: 15.05.2009
(51) Int. Cl.: A23L 5/20, A23L 33/10, A23L 33/15, A61L 33/16, A23L 33/17, A61K 9/08, A61K 9/10, A61K 9/48, A61K 8/04, A61P 43/00, A61K 9/20, A61Q 19/00, A61K 45/00

(54) **COMBINATION OF DRUGS HAVING DIFFERENT PHYSICAL PROPERTIES INTO SINGLE DOSAGE FORM**
KOMBINATION VON ARZNEIMITTELN MIT UNTERSCHIEDLICHEN PHYSIKALISCHEN EIGENSCHAFTEN IN EINER EINZELNEN DOSIERFORM
COMBINAISON DE MÉDICAMENTS AYANT DIFFÉRENTES PROPRIÉTÉS PHYSIQUES EN UNE SEULE FORME PHARMACEUTIQUE

(30) Priority: 15.05.2008 JP 2008128005
(43) Date of publication of application: 23.03.2011
(73) Proprietor: Cocokara fine Healthcare Inc., Kohoku-ku Yokohama-shi Kanagawa 222-0033 (JP)
(72) Inventor: FUJII, Takeru, Kobe-shi Hyogo 650-0047 (JP); HAYAKAWA, Eiji, Kobe-shi Hyogo 650-0047 (JP); HIRATA, Akihiko, Kobe-shi Hyogo 650-0047 (JP)
(74) Representative: Denison, Christopher Marcus
(86) International application number: PCT/JP2009/059393
(87) International publication number: WO 2009/139506

(56) References cited:
- EP-A1- 1 731 139
- EP-A1- 1 785 131
- WO-A1-2005/094789
- WO-A1-2006/025583
- WO-A2-2007/045689
- JP-A- 2002 301 357
- JP-A- 2004 008 837
- JP-A- 2004 008 837
- JP-A- 2004 043 355
- TOORISAKA: "Hypoglycemic effect of surfactant-coated insulin solubilized in a novel solid-in-oil-in-water (S/O/W) emulsion", INTERNATIONAL JOURNAL OF PHARMACEUTICS, ELSEVIER BV, NL, no. 252, 1 January 2003 (2003-01-01), pages 271-274, XP009107467, ISSN: 0378-5173, DOI: 10.1016/S0378-5173(02)00674-9
- EIICHI TOORISAKA ET AL: "Hypoglycemic effect of surfactant-coated insulin solubilized in a novel solid-in-oil-in-water (S/O/W) emulsion", INTERNATIONAL JOURNAL OF PHARMACEUTICS, ELSEVIER BV, NL, vol. 252, no. 1/2, 1 January 2003 (2003-01-01), pages 271-274, XP008146606, ISSN: 0378-5173, DOI: 10.1016/S0378-5173(02)00674-9
- PIAO H ET AL: "Oral delivery of diclofenac sodium using a novel solid-in-oil suspension", INTERNATIONAL JOURNAL OF PHARMACEUTICS, ELSEVIER BV, NL, vol. 313, no. 1-2, 26 April 2006 (2006-04-26), pages 159-162, XP027972292, ISSN: 0378-5173 [retrieved on 2006-04-26]
- HONGYU PIAO ET AL: "A Novel Solid-in-oil Nanosuspension for Transdermal Delivery of Diclofenac Sodium", PHARMACEUTICAL RESEARCH, KLUWER ACADEMIC PUBLISHERS-PLENUM PUBLISHERS, NL, vol. 25, no. 4, 25 September 2007 (2007-09-25), pages 896-901, XP019613044, ISSN: 1573-904X
- EIICHI TOORISAKA ET AL. INTERNATIONAL JOURNAL OF PHARMACEUTICS vol. 252, no. 1/2, 2003, pages 271 - 274, XP008146606
- HONGYU PIAO PHARMACEUTICAL DEVELOPMENT AND TECHNOLOGY vol. 12, no. 3, 2007, pages 321 - 325, XP008146609

## Description

### Technical Field

The present invention relates to a technique of combining two or more types of drugs or pharmaceutical additives exhibiting different physical properties into a single dosage form by means of nanodispersion of water-soluble drugs or low-solubility drugs and pharmaceutical additives having various physical properties, such as stabilizers or antioxidants, into the same dispersion medium.

### Background Art

In the treatment of diseases, the single-agent administration of a pharmaceutical product is rare. In most cases, it is usual to administer a plurality of therapeutic agents in combination depending on the stage of treatment. In the case of the surgery, for example, antibiotics or other substances are often administered through a site port at the time of introduction of a saccharide or amino acid infusion or the like through an intravenous line for the purpose of improving recovery, preventing shock, and preventing infection at the preoperative, perioperative, or postoperative phase.

When performing instillation or drug administration through a site port, it is essential that pharmaceutical products be solubilized. In addition, it is preferable that pharmaceutical products be solubilized at around a neutral state. Insoluble drugs cannot be administered, and administration of drugs that are soluble but become insolubilized at around a neutral state is very problematic from the viewpoint of treatment.

Including drugs that become insolubilized at around a neutral state, there are many low-solubility drugs as described above, and it is very difficult to formulate such drugs into injection preparations. In order to dissolve drugs having different physical properties; i.e., water-soluble drugs and low-solubility drugs, in water, it is essential to add a surfactant to prepare an emulsion. Not only does an emulsion become disintegrated via heat-induced swelling-the emulsion is disadvantageously separated into two phases; i.e., a precipitate and a clear solution, with the elapse of time. Thus, adjustment of an emulsion is very difficult.

The most apparent example of a substance comprising drugs having different physical properties is a nutritional factor that is extensively used in items ranging from pharmaceutical products to food or cosmetic products. In the case of coenzyme vitamins, for example, there are low-soluble (i.e., fat-soluble) vitamins, such as vitamin A, which causes night blindness, vitamin D, which promotes absorption of calcium, and vitamin E, which has vasodilator action, in addition to water-soluble vitamins, such as vitamin B1, which is known as a causal factor for beriberi and vitamin C, which causes scorbutus.

Among the 20 types of amino acids that are necessary for living organisms, 8 types thereof are lipophilic amino acids, and other types are hydrophilic amino acids. Among the 9 types of amino acids that cannot be synthesized *in vivo*, in particular, 6 types of amino acids (i.e., valine, leucine, isoleucine, methionine, phenylalanine, and tryptophan), except for hydrophilic lysine, threonine, and histidine, are lipophilic. Thus, it is obvious that a nutritional factor comprises two types of substances showing different properties.

Thus, there are actually two different types of "drugs" comprising paramedical products such as nutritional factors in addition to pharmaceutical products; i.e., water-soluble drugs and low-solubility drugs. This imposes a serious problem when preparing liquid preparations, such as injection preparations, infusion solutions, oral liquid preparations, lotions, poultices, and tapes or dosage forms using water-soluble bases, or when preparing oil injection preparations, oil capsules, oil external preparations, or plaster preparations.

For example, external preparations, such as lotions, poultices, tapes, oil external preparations, or plaster preparations, are applied or adhered to the skin. In the past, it was impossible to combine drugs having different physical properties into a single dosage form so as to induce functions of the drugs, when preparing such preparations. Specifically, skin, which serves as the ultimate barrier of a living organism, is a very lipophilic tissue, which is apparent from the fact that external preparations, such as water-soluble pharmaceutical products or water-soluble vitamins, which are used as paramedical products, have not been developed in the past.

Specifically, the human skin is a physiologically regenerated tissue in which corneocytes of keratinocytes are metabolized into cuticles with a period of 28 days in general. Thus, such epidermic tissue can be cultured and multiplied regardless of age, if necessary nutrition is provided. As long as the human skin tissue receives sufficient nutritional support from the blood, normal skin regeneration should be observed. However, various types of skin diseases are still observed, including atopy and psoriasis, because of the impossibility of applying functional water-soluble vitamins or other substances in the medical field as external preparations.

There are various causes for nutritional disorders of the skin. For example, nutritional deficiency of the skin resulting from low blood pressure, microvessels, or blood stasis is considered to result in development of skin disease due to the difference in differentiation speed between the cuticle layer and the keratinocyte layer, without regeneration of normal skin tissue. In order to resolve such problem, the blood flow must be increased with the aid of a vasodilator, or an external preparation comprising fat-soluble vitamin E exhibiting similar effects must be applied to the skin.

In fact, it is preferable to carry out percutaneous administration that directly delivers nutritional factors to the dermal tissue through the horny layer. However, the nature of the dermal tissue is that of a lipophilic barrier, and thus, water-soluble vitamins or amino acids would not be absorbed, and what is available is limited to external preparations of low-solubility (fat-soluble) vitamins.

Accordingly, administration of nutritional components, such as water-soluble vitamins or amino acids, that are necessary for the dermal tissue (i.e., the nutritional factors) must be carried out by incorporating such components into an external preparation as a derivative with high fat solubility or via an oral route. Thus, development of a technique that makes the surfaces of water-soluble vitamins fat-soluble without preparation thereof in the form of a derivative and combines such water-soluble vitamins into a single dose of oil base with fat-soluble vitamins has been awaited.

When production of pharmaceutical preparations or products of nutritional factors such as coenzymes including vitamins, or amino acids is intended, differences in physical properties, such as water solubility or low solubility, stability, taste, flavor, and the like are problematic when developing pharmaceutical agents or products. The term "coenzymes including vitamins" refers to coenzymes that are necessary for an enzyme to exhibit its activity *in vivo.* Deficiency thereof appears as a dysfunction of the metabolic system that is associated with an enzyme that utilized "coenzymes including vitamins" as a coenzyme.

Vitamins are classified into water-soluble vitamins that are easily soluble in water and fat-soluble vitamins that are easily soluble in fat. Water-soluble vitamins are further classified into the vitamin B family (vitamins B1, B2, B3, B5, B6, B7, B9, and B12) and vitamin C. Fat-soluble vitamins are further classified into vitamin A, vitamin D, vitamin E, and vitamin K. Vitamin B1 is also referred to as thiamine, vitamin B2 is also referred to as riboflavin, vitamin B3 is also referred to as niacin, vitamin B5 is also referred to as pantothenic acid, vitamin B6 is also referred to as pyridoxal, vitamin B7 is also referred to as biotin, vitamin B9 is also referred to as folic acid, vitamin B12 is also referred to as cyanocobalamin, and vitamin C is also referred to as ascorbic acid.

Examples of vitamin-based nutritional factors include a variety of vitamin derivatives and coenzymes, in addition to such vitamins. Examples of vitamin A include a variety of carotenoids, examples of vitamin D include a variety of calciferols, examples of vitamin E include a variety of tocopherols, and examples of vitamin K include a variety of naphthoquinone derivatives. Examples of coenzymes other than vitamins include water-soluble orotic acid and pangamic acid, amygdalin, methylmethionine sulfonium chloride, and carnitine. Examples of fat-soluble coenzymes include essential fatty acids, such as linolic acid, quercetin, and quinone coenzymes, such as ubiquinone. Ubiquinone is also referred to as coenzyme Q10 (CoQ10).

Amino acids, minerals, and saccharides are classified as water-soluble nutritional factors. A lipid is classified as a fat-soluble nutritional factor. Examples of lipids include a variety of polyphenols, seed oil extracts, sesamins, eicosapentaenoic acids, and propolis.

As described above, water-soluble and fat-soluble nutritional factors exist, and it is preferable that such components be used in adequate combination in accordance with the intended purpose of use.

When preparing injection preparations, infusion solutions, liquid preparations for internal use, eye drops, nasal drops, or aqueous lotions, water-soluble components of two or more components having different physical properties are dissolved in a water-soluble base without modification, the surface properties of low-solubility components are converted to achieve hydrophilicity, and such components are combined into a single dosage form as a drug-surfactant composite. By modifying one of the two water-soluble components, which changes its composition in a water soluble solvent, into the form of a hydrophilic drug-surfactant composite, direct contact between two components is prevented. This can prevent changes in drug compositions.
Non-Patent Document 1: Folia Pharmacol. Jpn., 127; 213-216,2006
Non-Patent Document 2: Proceedings of the 36th SPG Forum, 50-53,2001
Non-Patent Document 3: International Journal of Pharmaceutics 252; 271-274 (2003)
Non-Patent Document 4: International Journal of Pharmaceutics 338; 174-179 (2007)
Non-Patent Document 5: Pharmaceutical Development and Technology 12; 321-325 (2007)
Patent Document 1: JP Patent Publication (kokai) No. 2004-43355 A
Patent Document 2: WO 2005/094789
Patent Document 3: WO: 2006/025583
Patent Document 4: JP Patent Publication (kokai) No. 2004-8837 A
Additional relevant prior art includes EP1785131.

### Summary of the Invention

Pharmaceutical products or "paramedical products" such as nutritional factors used for pharmaceutical, food, or cosmetic products and pharmaceutical additives are divided into water-soluble substances and low-solubility substances. It is difficult to disperse water-soluble substances into an oil base comprising low-solubility substances dissolved therein. Also, it is very difficult to homogeneously disperse and incorporate low-solubility substances in an aqueous injection solution or liquid preparations for internal use comprising water-soluble substances.

In addition, it is very difficult to combine a plurality of drugs into the same dispersion medium depending on whether or not "drugs;" i.e., pharmaceutical products, paramedical products, and pharmaceutical additives used for producing such pharmaceutical products or paramedical products, are water-soluble or low-solubility drugs. The present invention, which is defined by the claims, is an artifice for dispersing and combining at least two types of drugs having different physical properties into a single dispersion medium by homogenizing physical properties of a plurality of drugs to be combined. Further, the present invention is intended to prepare a drug-surfactant composite comprising either or both of a plurality of drugs having different physical properties, such as water solubility or low solubility, that is an S/W, S/O, or S/O/W enclosed by surfactant for the purpose of inhibition of changes in drug compositions, improvement in stability and absorption in an organism, suppression of side effects, and masking of bitterness, acridness, or odor, thereby providing pharmaceutical products or paramedical products nanodispersed either in a water-soluble or oil base dispersion medium in accordance with the intended purpose.

In particular, pharmaceutical products or paramedical products comprising water-soluble vitamins or amino acid do not originally have percutaneous absorbability. By enclosing such hydrophilic pharmaceutical products and paramedical products with a surfactant, the physical properties of a drug-surfactant composite are converted to result in fat soluble so as to stabilize drugs and enhance percutaneous absorbability. Further, development of pharmaceutical products and paramedical products comprising at least one fat-soluble pharmaceutical product or paramedical raw material is awaited, and such pharmaceutical or paramedical products can be used as oil-base pharmaceutical preparations. For example, development of a technique that converts the surface of water-soluble vitamin C into a fat-soluble surface and combines the resultant with fat-soluble vitamin E to prepare a single dose of an oil-base preparation has been awaited. By producing a single dose of an oil-base preparation by converting the surface of water-soluble vitamin C into a fat-soluble surface, converting the surface of water-soluble hydroxyproline into a fat-soluble surface, and combining such substances together, the achievement of synergistic effects on skin, such as restoration of skin fitness and wound healing, has been expected.

The present invention has attained such objectives.

Further, production of a lotion, a liquid for electric induction, or a gel comprising an anionic, highly hydrophilic surfactant in the forms of injection preparations, infusion solutions, liquid preparations for internal use, water-soluble gel for internal use, or external gel creams has been expected through production of a drug-surfactant composite comprising low-solubility drugs enclosed by a hydrophilic surfactant. For example, gels for internal use comprising low-solubility ubiquinone dissolved in a water-soluble gel through conversion of the surface of low-solubility ubiquinone into a hydrophilic surface have been desired. In addition, a preparation that restores skin fitness when dissolved in an electrode aqueous solution for iontophoresis has been desired. The present invention has attained such objectives.

By converting drug surfaces to hydrophilic and fat-soluble surfaces to homogenize surface properties of a plurality of drugs according to need, water-soluble or fat-soluble pharmaceutical preparations can be produced. By rendering the surfaces hydrophilic, drugs can be ionized, and drugs can be solubilized in a water-soluble solvent.

Since two or more types of drugs having different physical properties can be stably combined in one dispersion medium, uneven drug contents resulting from suspension of powders, precipitation or phase separation resulting from heating, and aging variation over time due to storage can be prevented. In addition, this technique is capable of preventing direct contact of drugs, which causes changes in drug compositions, and thus is capable of inhibiting changes in drug compositions.

Examples of changes in drug compositions include an addition reaction between vitamin C and sodium pyrosulfife, an amino carbonyl reaction between amino acid or an amino-group-containing drug and a saccharide or aldehyde-group-containing drug, a precipitation reaction between a compound having a sulfuric acid group and a drug having an amino group, and changes in drug composition of terbinafine hydrochloride and benzalkonium chloride. Other examples include changes in drug compositions of polyethylene glycol and phenobarbital and of polyethylene glycol and a vinca alkaloid drug, changes in drug compositions of dibutyl hydroxytoluene and an amine-containing compound, Schiff base formation involving aminophylline and glucose, the furfural reaction involving tetracycline and alkaline earth, racemization of epinephrine by a sulfurous acid group, acid amide formation involving benzocaine and citric acid, formation of a gentamicin-carbenicillin composite, aminolysis involving aspirin and phenylephrine, and epimerization involving multivitamin preparations. Development of techniques that overcome such drawbacks has been awaited. Thus, the method of the present invention comprising coating a surface (or surfaces) of one component (or both components) with a surfactant to prepare a drug-surfactant composite has enabled inhibition of changes in drug composition.

Examples of combinations of low-solubility drugs and water-soluble drugs, low-solubility drugs and other low-solubility drugs, and water-soluble drugs and other water-soluble drugs into a single dosage form for the purpose of enhancing the effects of drugs or inhibiting side effects include a combination of eicosapentaenoic acid and Lipitor (i.e., therapeutic agents for hyperlipidemia), a combination of vitamin K2 and lansoprazole (i.e., a proton pump inhibitor), a combination of a calcium antagonist and an ACE inhibitor, a combination of haloperidol decanoate and haloperidol, a combination of vitamin E and vitamin C, a combination of vitamin A and vitamin C, a combination of ascorbyl stearate and ascorbic acid or magnesium ascorbyl phosphate, a combination of coenzyme Q10 and magnesium ascorbyl phosphate, and a combination of indomethacin and diclofenac sodium. When such components were combined as liquid agents according to conventional techniques, however, one of the drugs was precipitated, suspended, aggregated, or underwent changes in drug composition with pharmaceutical additives when either a water-soluble or fat-soluble solvent was used. Thus, it was impossible to develop pharmaceutical preparations exhibiting high content uniformity. According to the method of the present invention, a surface (or surfaces) of one component (or both components) was (or were) coated with a surfactant to prepare a drug-surfactant composite, and such components were combined into a single dose.

In order to overcome the above-described drawbacks, accordingly, the present invention is intended to produce a drug-surfactant composite comprising drugs enclosed by a surfactant and produce an S/W preparation or product comprising drug components microdispersed in a hydrophilic solvent, an S/O preparation or product comprising drug components dispersed in a fat-soluble solvent, or an S/O/W preparation or product comprising an S/W and S/O preparation or a product dispersed in a hydrophilic solvent. Thus, a plurality of drug components were combined in a single dispersion medium. Examples of dispersion media include hydrophilic, water-soluble, and fat-soluble solvents.

This description refers to the contents as disclosed in the description and/or drawings of Japanese Patent Application No. 2008-128005, which is a priority document of the present application.

### Brief Description of the Drawings

Fig. 1 shows a method for producing a drug-surfactant composite.
Fig. 2 shows a drug-surfactant composite, Solid (S).
Fig. 3 shows Solid-in-Water (S/W).
Fig. 4 shows Solid-in-Oil (S/O).
Fig. 5 shows Solid-in-Oil-in-Water (S/O/W).
Fig. 6 shows stabilization of VC·PMg via preparation of an S/O preparation. The photograph was stored at room temperature for 2 years, the photograph shows, from left to right, a dispersion of S/O VC·PMg:VC·PMg-LipoS in isopropyl myristate (Production Example 5), a similar hue from a dispersion medium of a negative control, browning of a VC·PMg solution, a VC·PMg aqueous solution of a positive control, and the ratio of dispersion medium (Oil) : isopropyl myristate in an S/O preparation as a negative control.
Fig. 7 shows an improvement in skin absorption of vitamin C via preparation of an S/O preparation.

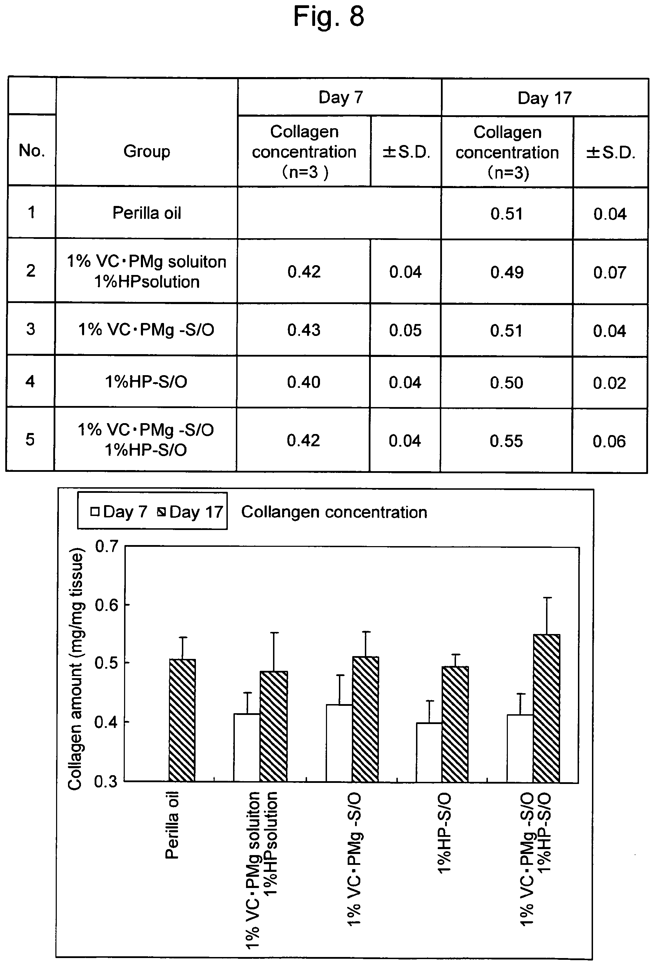

Fig. 8 shows changes in collagen content in the dermal tissue of a rat back with the elapse of time when VC·PMg and HP were applied (i.e., the results of assay of collagen content in the dermal tissue of the rat back).

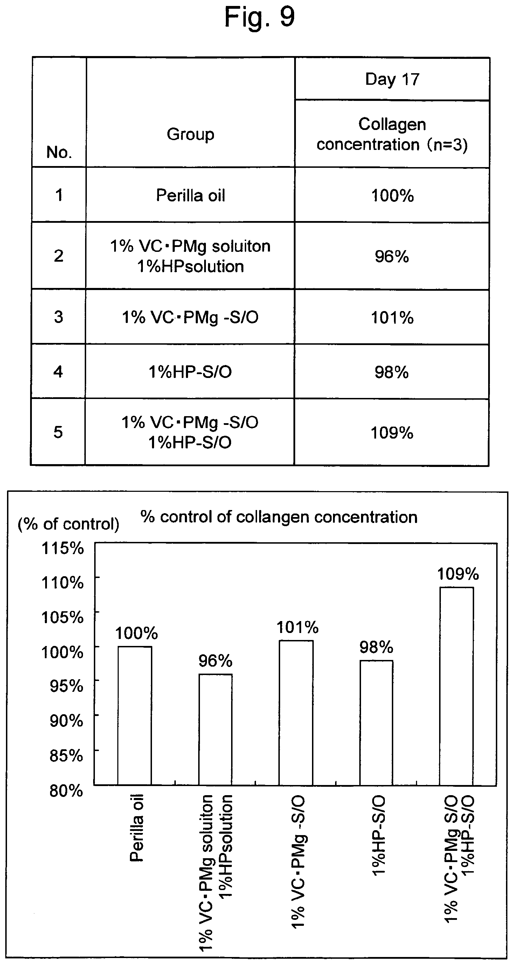

Fig. 9 shows an increase in collagen content on day 17 resulting from combination of a plurality of S/O preparations (i.e., collagen concentration relative to perilla oil as 100%).

### Best Modes for Carrying out the Invention

### 1. Introduction

The present invention concerns a technique, defined by the claims, for preparing a drug-surfactant composite comprising a plurality of drugs exhibiting different physical properties (the term "drugs" also refers to compounds or substances), that is an S/W, S/O, or S/O/W enclosed by surfactant in accordance with the intended purpose, in order to modify surface properties to hydrophilic to lipophilic or vice versa and to combine a plurality of drugs into a single dose. This can enhance chemical and physical stability, enhance absorbability, reinforce pharmacological effects, and reduce side effects. In addition, bitterness, acridness, odor, and other problems can be modified. The present invention is intended to provide a pharmaceutical product or paramedical product comprising two or more drugs having different physical properties in a single dosage form, in which at least one drug is a drug-surfactant composite.

Also, the present invention is intended to provide a product comprising a drug-surfactant composite, such as a pharmaceutical preparation or paramedical product, which can comprise two or more types of drugs having different physical properties or drug-surfactant composites in a single dispersion medium (e.g., an aqueous or fat-soluble solvent) by preparing a drug-surfactant composite comprising drugs enclosed by a surfactant. It is intended to homogenize physical properties of drugs, microdisperse drugs in a hydrophilic or fat-soluble solvent, stabilize drugs, enhance absorbability, and mask bitterness, acridness, or odor.

When dispersing water-soluble drugs in a fat-soluble solvent, water-soluble drugs are nanocoated with a lipophilic surfactant to prepare an S preparation having a fat-soluble surface (hereafter, it is referred to as lipophilic S or LipoS). Alternatively, this LipoS is dispersed in a fat-soluble solvent to prepare an S/O preparation. LipoS or S/O prepared by converting the surfaces of water-soluble drugs to fat-soluble surfaces is dispersed in a fat-soluble solvent, and the resultant can be combined with fat-soluble drugs.

When two types of water-soluble drugs are dissolved or dispersed in an aqueous solvent (it may be referred to as a hydrophilic solvent), such two types of drugs can be dissolved or dispersed without a reaction occurring upon direct contact. For example, one of the water-soluble drugs is prepared in an LipoS form as described above, the resultant is then prepared in an S/O form, the resultant is microdispersed in a hydrophilic solvent to prepare S/O/W, and contact thereof with other water-soluble drugs can be prevented in a water-soluble solvent.

When dispersing lipophilic, low-solubility drugs in a hydrophilic solvent, low-solubility drugs are nanocoated with a hydrophilic surfactant to prepare an S preparation having a hydrophilic surface (hereafter, it is referred to as hydrophilic S or HydroS). This HydroS is dispersed in a hydrophilic solvent to prepare S/W.

Further, hydrophilic, low-solubility drugs are nanocoated with a lipophilic surfactant to prepare an S preparation having a fat-soluble surface (hereafter, it is referred to as lipophilic S or LipoS). Alternatively, LipoS is dispersed in a fat-soluble solvent to prepare S/O. LipoS or S/O is dispersed in a fat-soluble solvent, and the resultant can be combined with other fat-soluble drugs.

Examples of hydrophilic solvents include hydrophilic gel and a hydrophilic gel base, and physical properties can be altered via gelation. This enables LipoS to be dissolved in hydrophilic gel as well as HydroS.

The term "disperse" used herein also means "dissolve."

### 2. Drugs

Examples of drugs include pharmaceutical products, paramedical products, nutritional factors, and pharmaceutical additives. Pharmaceutical products are substances having pharmacological activity and therapeutic, diagnostic, or preventive activity. Specific examples include antibiotics, antiviral agents, anesthetics, steroid drugs, antiinflammatory agents, antitumor agents, antigens, vaccines, antibodies, decongestants, hypotensive drugs, sedatives, birth-control drugs, agents for promoting pregnancy, anticholinergic agents, analgesics, antidepressants, antipsychotics, diuretics, cardiovascular agents, vasoactive agents, nonsteroidal anti-inflammatory drugs, and nutrient preparations. Examples include: antiviral agents such as acyclovir; steroidal anti-inflammatory agents such as fluorometholone, dexamethasone, and prednisolone, and non-steroidal anti-inflammatory agents such as indomethacin and diclofenac sodium; hypotensive agents such as griseofulvin, nifedipine, and nicardipine; anticancer agents such as paclitaxel, Adriamycin, docetaxel, daunomycin, methotrexate, mitomycin C, camptothecin, taxol, vincristine, and derivatives of any thereof; macrolide antibiotics such as Ilotycin, erythromycin, and clarithromycin; antifungal agents such as amphotericin B, itraconazole, and miconazole; hormones such as estradiol, testosterone, progesterone, diethylstilbestrol, derivatives of any thereof, growth hormones, and insulin; prostaglandin or prostacycline agents such as Cilostazol; bone metabolic turnover agents, such as vitamin D, bisphosphonate, and calcitonin; therapeutic agents that act on the blood, such as G-CSF and erythropoietin; immunomodulators, such as ciclosporin and FK506; and agents for gastrointestinal functions, such as omeprazole and sucralfate. Further examples include a therapeutic agent for polakisuria, an antiemetic, an anti-migraine agent, an anti-dementia agent, an antiparkinsonism agent, an anti-hyperlipidemia agent, an anti-asthma agent, a therapeutic agent for atopic dermatitis, a therapeutic agent for psoriasis, an antirheumatic agent, a therapeutic agent for leukoplakia, and an agent for preventing imminent abortion. Furthermore, pharmaceutical products of a low-molecular-weigh compound, an organometal compound, a saccharide, a nucleic acid, a protein, a peptide, a metal, and an immunological factor may be used.

Paramedical products can be selected from among amino acids, coenzymes including vitamins, minerals, lipids, and saccharides.

Further, nutritional factors can be selected from among amino acids, coenzymes including vitamins, minerals, lipids, and saccharides.

Examples of amino acids include: polar amino acids, such as lysine, arginine, histidine, threonine, glutamic acid, asparatic acid, and asparagine; neutral amino acids, such as glycine, alanine, glutamine, cysteine, taurine, proline, and serine; lipophilic amino acids, such as valine, leucine, isoleucine, methionine, phenylalanine, tryptophan, cystine, and tyrosine; and derivatives of any thereof. Examples include amino acid derivatives such as dipeptide and tripeptide derivatives.

Examples of vitamins include: aqueous vitamins, such as vitamin B1, vitamin B2, vitamin B5, vitamin B6, vitamin B12, niacin, vitamin C, folic acid, biotin, and carnitine; fat-soluble vitamins, such as vitamin A, vitamin D, vitamin K, and vitamin E; and derivatives of any thereof. In addition, examples include coenzymes, such as ubiquinone.

Examples of minerals include calcium, iron, phosphorus, magnesium, sodium, potassium, copper, iodine, manganese, selenium, zinc, chromium, and molybdenum.

Examples of lipids include: simple lipids, such as fat (neutral fat) and wax; complex lipids, such as glycolipids and phospholipids; and derivatives of any thereof. Further examples of lipids include lipid components extracted from animals or plants. Specific examples include various polyphenols, seed oil extracts, sesamins, eicosapentaenoic acids, and propolis.

Examples of saccharides include: monosaccharides, such as glucose and fructose; disaccharides, such as sucrose and maltose; polysaccharides, such as starch and glycogen; and derivatives of any thereof

Examples of pharmaceutical additives include stabilizers, antioxidants, colorants, antiseptics, preservatives, soothing agents, aroma chemicals, and buffers. Specific examples include sodium sulfite, sodium benzoate, tetrasodium edetate, benzalkonium chloride, sorbic acid, disodium glycyrrhizinate, glycyrrhetic acid, sodium salicylate, dibutyl hydroxytoluene, sodium thiomalate, trometamol, nicotinic acid amide, parabens, heparin sodium, meglumine, saccharin, crotamiton, allantoin, sodium hyaluronate, dimethylpolysiloxane, fennel oil, cinnamon oil, peppermint oil, vanillin, sodium copper chlorophyllin, cyclodextrin, melamine, phytic acid, ferulic acid, ethyl aminobenzoate, inositol, lidocaine hydrochloride, creatinine, adipic acid, and urea.

Such drugs are classified as water-soluble drugs or low-solubility drugs in accordance with their affinity for water.

### 3. Low solubility

Classification of water-soluble drugs and low-solubility drugs is indicated in the Biopharmaceutical Classification System (BCS). BCS is defined as follows in accordance with the guidance of the U.S. Food and Drug Administration (USFDA).

Guidance for industry: Waiver of *in vivo* bioavailability and bioequivalence studies for immediate-release solid oral dosage forms based on a biopharmaceutics classification system

In accordance with BCS, a drug is classified as a water-soluble drug when solubility in 250 ml of buffer over a pH range of 1.0 to 7.5 is equal to or greater than the maximal drug content in 1 unit of a preparation, and a drug is classified as a low-solubility drug when such solubility is equal to or lower than the maximal drug content.

Low-solubility drugs can be further classified as hydrophilic or lipophilic, low-solubility drugs in accordance with the hydrophilic-lipophilic balance.

### 4. Surfactant

Surfactants are classified as anionic, cationic, amphoteric, or nonionic surfactants. Surfactants comprise in their molecules hydrophilic groups and lipophilic groups, and surfactants are classified as hydrophilic or lipophilic surfactants, in accordance with the hydrophilic-lipophilic balance (i.e., the HLB value).

The hydrophilic-lipophilic balance of a surfactant is expressed using hydrophilic lipophilic balance (HLB) values as indicators. HLB values range from 0 to 20. An HLB value closer to 0 indicates higher lipophilic properties, and an HLB value closer to 20 indicates higher hydrophilic properties.

Hydrophilic surfactants generate O/W emulsions at an HLB value of approximately 8 or higher. Lipophilic surfactants generate W/O emulsions at an HLB value of less than approximately 8.

A lipophilic surfactant is used to generate an S/O emulsion. A hydrophilic surfactant in an amount that is half or less the amount of the lipophilic surfactant can be added. The amount is preferably 10% or less.

An S/O/W preparation obtained by dispersing an S/O oil in an aqueous phase is prepared by adding a hydrophilic surfactant to the aqueous phase. In this case, a lipophilic or hydrophilic surfactant may also be added to the oil phase in an amount that is half or less the amount of a hydrophilic surfactant to be added to the aqueous phase. The amount is preferably 10% or less. Also, a lipophilic surfactant may be added to the aqueous phase. The amount of a lipophilic surfactant to be added to the aqueous phase is half or less the amount of the hydrophilic surfactant, and the amount is preferably 10% or less.

A hydrophilic surfactant is used to generate an S/W emulsion. A lipophilic surfactant in an amount that is half or less the amount of the hydrophilic surfactant can be added. The amount is preferably 10% or less.

Examples of hydrophilic surfactants include anionic, cationic, amphoteric, and nonionic surfactants described below.

Examples of anionic surfactants that can be used include fatty acid soap, naphthenic acid soap, sulfuric acid ester of long-chain alcohol, polyoxyethylene alkylphenyl ether sulfuric acid ester salt, fatty acid monoglyceride sulfuric acid ester, fatty acid monoalkanolamide sulfuric acid ester, alkaline sulfonic acid salt, α-sulfo fatty acid salt, dialkyl sulfosuccinic acid salt, polyoxyethylene octylphenyl ether sulfonic acid salt, alkyl benzene sulfonic acid salt, polyoxyethylene alkylphenol ether phosphoric acid ester salt, polyoxyethylene alkyl ether phosphoric acid ester salt, and sodium lauryl sulfate.

Examples of cationic surfactants that can be used include long-chain primary amine salt, alkyltrimethylammonium salt, dialkyldimethylammonium salt, alkylpyridinium salt, polyoxyethylene alkylamine, and alkyl imidazoline.

Examples of amphoteric surfactants that can be used include N-alkyl β-aminopropionic acid salt and N-alkyl β-iminodipropionic acid salt.

Examples of hydrophilic nonionic surfactants that can be used include an ethylene oxide adduct of a higher alcohol, an ethylene oxide adduct of alkylphenol, an ethylene oxide adduct of fatty acid, an ethylene oxide adduct of polyhydric alcohol-fatty acid ester, an ethylene oxide adduct of higher alkylamine, an ethylene oxide adduct of fatty acid amide, an ethylene oxide adduct of an oil or fat, fatty acid ester of glycerin, fatty acid ester of pentaerythritol, alkyl ether of a polyhydric alcohol, and fatty acid amide of alkanolamines.

Among nonionic surfactant, for example, sorbitol and sorbitan fatty acid esters, polyoxyethylene sorbitan fatty acid ester, polyethylene glycol fatty acid ester, sucrose fatty acid ester, polyoxyethylene castor oil (polyethoxylated castor oil), polyoxyethylene hydrogenated castor oil (polyethoxylated hydrogenated castor oil), polyoxyethylene polypropylene glycol copolymer, fatty acid ester of glycerin, and polyglyceryl fatty acid ester are preferably used.

As polyoxyethylene sorbitan fatty acid ester, for example, polysorbates 20, 40, 60, and 80 are particularly preferable. As polyethylene glycol fatty acid ester, for example, polyethylene glycol monolaurate is particularly preferable. As sucrose fatty acid ester, for example, sucrose palmitic acid esters (e.g., trade name: P-1670, Mitsubishi-Kagaku Foods Corporation), sucrose stearic acid esters (e.g., trade name: S-1670, Mitsubishi-Kagaku Foods Corporation), and sucrose lauric acid esters (e.g., trade name: L-1695, Mitsubishi-Kagaku Foods Corporation) are particularly preferable. As polyoxyethylene castor oil (polyethoxylated castor oil), for example, polyoxyethylene glycerol triricinoleate 35 (Polyoxy 35 Castor Oil; trade name: Cremophor EL or EL-P, BASF Japan Ltd.) is particularly preferable. As polyoxyethylene hydrogenated castor oil (polyethoxylated hydrogenated castor oil), for example, polyoxyethylene hydrogenated castor oil 50 and polyoxyethylene hydrogenated castor oil 60 are particularly preferable. As a polyoxyethylene polyoxypropylene glycol copolymer, for example, polyoxyethylene (160) polyoxypropylene (30) glycol (trade name: Adeka Pluronic F-68, Adeka Corporation) is particularly preferable. As polyglyceryl fatty acid ester, for example, decaglycerol monolaurate (Decaglyn 1-L, Nikko Chemicals Co., Ltd.) is preferable.

Examples of lipophilic surfactants include: sucrose fatty acid esters, such as sucrose stearic acid ester, sucrose palmitic acid ester, sucrose oleic acid ester, sucrose lauric acid ester, sucrose behenic acid ester, and sucrose erucic acid ester; sorbitan fatty acid esters, such as sorbitan monostearate, sorbitan tristearate, sorbitan monooleate, sorbitan trioleate, and sorbitan sesquioleate; fatty acid esters of glycerin, such as glycerol monostearate and glycerol monooleate; and polyglyceryl fatty acid esters, such as diglyceryl tetraisostearate, diglyceryl diisostearate, and diglyceryl monoisostearate.

Drug-surfactant composites are Solid-in-Water (S/W) preparations, Solid-in-Oil (S/O) preparations, or Solid-in-Oil-in-Water (S/O/W) preparations, and products produced therefrom are pharmaceutical products or paramedical products such as food (supplement) or cosmetic products.

### 5. Drug-surfactant composite

The drug-surfactant composites of the present invention, which is defined by the claims, can be prepared in the form of products comprising any of four types of drug-surfactant composites: 1) a drug-surfactant composite (Solid) comprising drugs enclosed by a surfactant, which is used in such state; 2) a Solid-in-Water (S/W) preparation comprising a drug-surfactant composite (Solid) dispersed or dissolved in a hydrophilic solvent; 3) a Solid-in-Oil (S/O) preparation comprising a drug-surfactant composite (Solid) dispersed or dissolved in a fat-soluble solvent; and 4) a Solid-in-Oil-in-Water (S/O/W) preparation comprising S/O dispersed or dissolved in a hydrophilic solvent.

A drug-surfactant composite can be in a variety of forms in accordance with the intended purpose of a product. When S is powder, for example, it is used in a solid state. When S is a liquid or slurry, such liquid or slurry is adsorbed with the aid of an additive that can be used for a pharmaceutical, food, or cosmetic product to solidify the liquid or slurry in the form of powder.

When S is prepared into the form of a liquid product, including a gel, a cream, and an ointment, similarly, the drug-surfactant composite can be in any of three different forms: i.e., S/W comprising S dispersed in a hydrophilic solvent, S/O comprising S dispersed in a fat-soluble or lipophilic solvent, or S/O/W comprising S/O dispersed in a hydrophilic solvent.

The drug-surfactant composite of the present invention comprises drugs enclosed by a surfactant, resulting in stabilization of drugs, enhancement of absorbability, and masking of bitterness and other problems, for the purpose of improvement in affinity for water or dispersibility of drags in a fat-soluble liquid agent.

Embodiments of the drug-surfactant composite (S) of the present invention, which is defined by the claims, and products containing the drug-surfactant composite are described in detail.

### 5-1. Drug-surfactant composite (S)

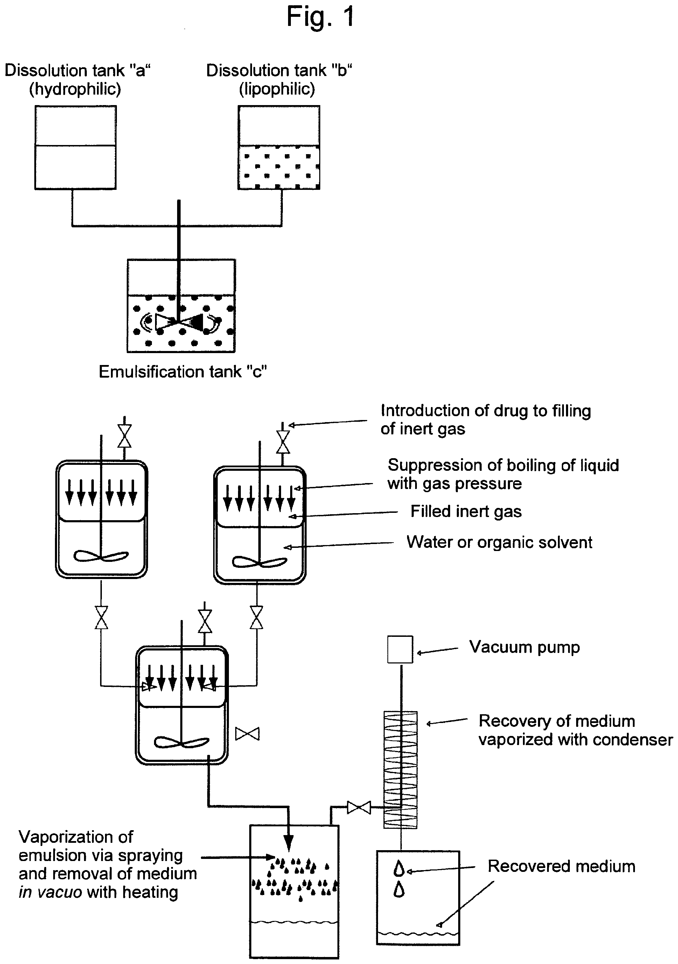

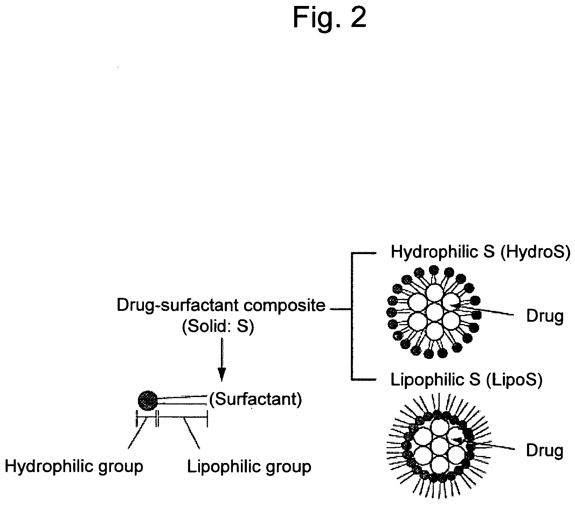

As shown in Fig. 1, the drug-surfactant composite (S) is prepared by dissolving or dispersing drugs and surfactants in a dissolution tank "a" comprising a hydrophilic solvent or a dissolution tank "b" comprising a fat-soluble solvent, mixing and emulsifying the resultant in an emulsification tank "c," and subjecting the resultant to vacuum dehydration or lyophilization for removal of moisture or a solvent. The structure is as shown in Fig. 2.

When drugs are lipophilic, low-solubility drugs, the surfactant encloses the low-solubility drugs with lipophilic groups thereof facing inward and hydrophilic groups thereof facing outward, and low-solubility drugs are modified into the form of hydrophilic, low-solubility drugs. Hereafter, the resultant is abbreviated as "hydrophilic S" or "HydroS."

When drugs are hydrophilic, low-solubility drugs, the surfactant encloses the low-solubility drugs with hydrophilic groups thereof facing inward and lipophilic groups thereof facing outward, and low-solubility drugs are modified into the form of fat-soluble drugs. Hereafter, the resultant is abbreviated as "lipophilic S" or "LipoS."

When drugs are water-soluble drugs, the surfactant encloses the water-soluble drugs with hydrophilic groups thereof facing inward and lipophilic groups thereof facing outward, and water-soluble drugs (which may be referred to as "hydrophilic drugs") are modified into the form of fat-soluble drugs. Hereafter, the resultant is abbreviated as "lipophilic S" or "LipoS."

### 5-1-1. More specifically, HydroS or LipoS can be produced by a method comprising:

(I) a step of dissolving and/or dispersing drugs in an aqueous solution or organic solvent to obtain a solution and/or dispersion;
(II) a step of liquefying and dissolving a lipophilic or hydrophilic surfactant via heating or other means or dissolving and/or dispersing a lipophilic or hydrophilic surfactant in an organic solvent to obtain a solution and/or dispersion;
(III) a step of mixing the solution and/or dispersion obtained in step (I) with the solution and/or dispersion obtained in step (II) to obtain an emulsion or dispersion; and
(IV) a step of removing a solvent or moisture from the emulsion or dispersion obtained in step (III).

(Step (I) and step (II) may be carried out in any order, and these steps may be carried out simultaneously. In the above-described method, further, drugs may be added in the form of fine powder in step (II) to obtain a solution or dispersion. Water may further be added thereto to obtain an emulsion.)

### 5-1-2. Steps for producing LipoS are first described in greater detail.

### Step (I) Dissolution and/or dispersion of drugs in aqueous solution

Drugs can be dissolved in water with the use of a solubilizer. Examples of solubilizers for drugs include water-soluble acids such as citric acid, adipic acid, lactic acid, phosphoric acid, and carbonic acid compounds, water-soluble bases such as sodium hydroxide, ammonium hydroxide, and sodium phosphate compounds, clathrate compounds of amino acids, proteins, urea, and cyclodextrin, various types of nucleic acids, and various types of saccharides. Such solubilizers can be used alone or in combinations of two or more. Heating and/or pressurization can also be carried out in order to efficiently dissolve or disperse drugs in water.

### Step (II) Dissolution or dispersion of lipophilic surfactant

When producing LipoS, a lipophilic surfactant having an HLB value of 0 to less than 8 can be used. Examples of lipophilic surfactants include: sucrose fatty acid esters, such as sucrose stearic acid ester, sucrose palmitic acid ester, sucrose oleic acid ester, sucrose lauric acid ester, sucrose behenic acid ester, and sucrose erucic acid ester; sorbitan fatty acid esters, such as sorbitan monostearate, sorbitan tristearate, sorbitan monooleate, sorbitan trioleate, and sorbitan sesquioleate; fatty acid esters of glycerin, such as glycerol monostearate and glycerol monooleate; and polyglyceryl fatty acid esters, such as diglyceryl tetraisostearate, diglyceryl diisostearate, and diglyceryl monoisostearate. Such surfactants are dispersed or dissolved in an organic solvent. Two or more types of such lipophilic surfactants may be used, and a hydrophilic surfactant can be added in an amount that is half or less the amount of the lipophilic surfactant. Such amount is preferably 10% or less.

### Step (III) Emulsification or dispersion

The aqueous solution comprising drugs dissolved and/or dispersed therein obtained in step (I) (hereafter, it may be referred to as the "aqueous solution of step (I)") is mixed with the organic solvent comprising a lipophilic surfactant dissolved and/or dispersed therein obtained in step (II) (hereafter, it may be referred to as the "organic solvent of step (II)"), and the mixture is emulsified. The aqueous solution of step (I) may be added to the organic solvent of step (II), and the organic solvent of step (II) may be added to the aqueous solution of step (I). Preferably, the aqueous solution of step (I) and the organic solvent of step (II) are introduced into an emulsification tank while being heated and pressurized.

### Step (IV) Removal of solvent and/or moisture

The step of removal of a solvent and/or moisture may be carried out in accordance with a conventional technique. Examples thereof include, but are not limited to, vacuum-freeze drying, drying under reduced pressure, and nitrogen purging. An optimal method of drying may be selected in accordance with the intended purpose.

The resulting S, a surfactant-drug composite, may be dispersed in water or a fat or oil by making use of the properties of surfactant. Also, such composite may be adsorbed to an adequate carrier such as silica and used for producing solid preparations, such as powders, granules, tablets, or capsules, as an active component.

### 5-1-3. Steps for producing HydroS are described as follows in greater detail.

### Step (I) Dissolution and/or dispersion of lipophilic drugs in organic solvent

Drugs can be dissolved in an organic solvent during heating and/or the application of pressure, in order to efficiently dissolve and/or disperse drugs in an organic solvent.

### Step (II) Dissolution or dispersion of hydrophilic surfactant

When producing HydroS, a surfactant having an HLB value of 8 or greater can be used. Examples include anionic, cationic, amphoteric, and nonionic surfactants described below.

Examples of anionic surfactants that can be used include fatty acid soap, naphthenic acid soap, sulfuric acid ester of long-chain alcohol, polyoxyethylene alkylphenyl ether sulfuric acid ester salt, fatty acid monoglyceride sulfuric acid ester, fatty acid monoalkanolamide sulfuric acid ester, alkaline sulfonic acid salt, α-sulfo fatty acid salt, dialkyl sulfosuccinic acid salt, polyoxyethylene octylphenyl ether sulfonic acid salt, alkyl benzene sulfonic acid salt, polyoxyethylene alkylphenol ether phosphoric acid ester salt, polyoxyethylene alkyl ether phosphoric acid ester salt, and sodium lauryl sulfate.

Examples of cationic surfactants that can be used include long-chain primary amine salt, alkyltrimethylammonium salt, dialkyldimethylammonium salt, alkylpyridinium salt, polyoxyethylene alkylamine, and alkyl imidazoline.

Examples of amphoteric surfactants that can be used include N-alkyl β-aminopropionic acid salt and N-alkyl β-iminodipropionic acid salt.

Examples of water-soluble nonionic surfactants that can be used include an ethylene oxide adduct of a higher alcohol, an ethylene oxide adduct of alkylphenol, an ethylene oxide adduct of fatty acid, an ethylene oxide adduct of polyhydric alcohol-fatty acid ester, an ethylene oxide adduct of higher alkylamine, an ethylene oxide adduct of fatty acid amide, an ethylene oxide adduct of an oil or fat, fatty acid ester of glycerin, fatty acid ester of pentaerythritol, alkyl ether of a polyhydric alcohol, and fatty acid amide of alkanolamines.

Among such nonionic surfactants, for example, sorbitol and sorbitan fatty acid esters, polyoxyethylene sorbitan fatty acid ester, polyethylene glycol fatty acid ester, sucrose fatty acid ester, polyoxyethylene castor oil (polyethoxylated castor oil), polyoxyethylene hydrogenated castor oil (polyethoxylated hydrogenated castor oil), polyoxyethylene polypropylene glycol copolymer, fatty acid ester of glycerin, or polyglyceryl fatty acid ester can be preferably used.

As polyoxyethylene sorbitan fatty acid ester, for example, polysorbates 20, 40, 60, and 80 are particularly preferable. As polyethylene glycol fatty acid ester, for example, polyethylene glycol monolaurate is particularly preferable. As sucrose fatty acid ester, for example, sucrose palmitic acid esters (e.g., trade name: P-1670, Mitsubishi-Kagaku Foods Corporation), sucrose stearic acid esters (e.g., trade name: S-1670, Mitsubishi-Kagaku Foods Corporation), and sucrose lauric acid esters (e.g., trade name: L-1695, Mitsubishi-Kagaku Foods Corporation) are particularly preferable. As polyoxyethylene castor oil (polyethoxylated castor oil), for example, polyoxyethylene glycerol triricinoleate 35 (Polyoxy 35 Castor Oil; trade name: Cremophor EL or EL-P, BASF Japan Ltd.) is particularly preferable.

As polyoxyethylene hydrogenated castor oil (polyethoxylated hydrogenated castor oil), for example, polyoxyethylene hydrogenated castor oil 50 and polyoxyethylene hydrogenated castor oil 60 are particularly preferable. As a polyoxyethylene polyoxypropylene glycol copolymer, for example, polyoxyethylene (160) polyoxypropylene (30) glycol (trade name: Adeka Pluronic F-68, Adeka Corporation) is particularly preferable. As polyglyceryl fatty acid ester, for example, decaglycerol monolaurate (Decaglyn 1-L, Nikko Chemicals Co., Ltd.) is preferable.

At least 1 type of such hydrophilic surfactant can be used, two or more types thereof can be used, and a lipophilic surfactant can be added in an amount that is half or less the amount of the hydrophilic surfactant. Such amount is preferably 10% or less.

### Step (III) Emulsification or dispersion

The organic solvent comprising drugs dissolved and/or dispersed therein obtained in step (I) (hereafter, it may be referred to as the "organic solvent of step (I)") is mixed with the aqueous solution comprising a hydrophilic surfactant dissolved and/or dispersed therein obtained in step (II) (hereafter, it may be referred to as the "aqueous solution of step (II)"), and the mixture is emulsified. The organic solvent of step (I) may be added to the aqueous solution of step (II), and the aqueous solution of step (II) may be added to the organic solvent of step (I). Preferably, the organic solvent of step (I) and the aqueous solution of step (II) are introduced into an emulsification tank while being heated and pressurized.

### Step (IV) Removal of solvent and/or moisture

When producing LipoS and HydroS, emulsification may be carried out with the use of, for example, a thin-film rotary high-speed agitator, a Hydromax mixer, an impeller agitator, or an Agi Homo mixer. That is, the air tightness of a shaft seal at the time of high-speed agitation while suppressing bumping with gas pressure is important, and a mechanical seal or the like is used.

### Step (IV) Removal of solvent and/or moisture

The step of removal of a solvent and/or moisture may be carried out in accordance with a conventional technique. Examples thereof include, but are not limited to, vacuum-freeze drying, drying under reduced pressure, and nitrogen purging. An optimal method of drying may be selected in accordance with the intended purpose.

The resulting S, a surfactant-drug composite, may be dispersed in water or a fat or oil by making use of the properties of the surfactant. Also, such composite may be adsorbed to an adequate carrier such as silica and used for producing solid preparations, such as powders, granules, tablets, or capsules, as an active component.

### 5-2. Solid-in-Water (S/W)

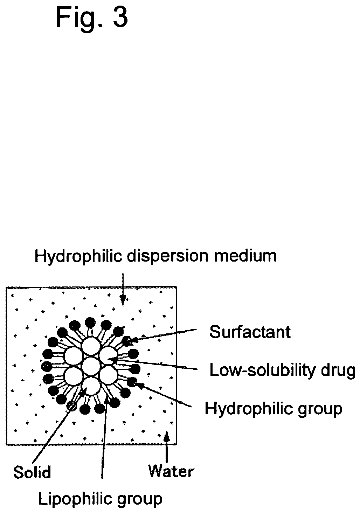

Drugs, and particularly lipophilic, low-solubility drugs, are dispersed and/or dissolved in a dissolution tank "a" or "b," a surfactant is dissolved in a dissolution tank "a" or "b," a particularly highly hydrophilic surfactant is dissolved in a dissolution tank "a", and the drugs and the surfactant are subjected to emulsification in an emulsification tank "c," followed by removal of moisture and a solvent, to prepare a drug-surfactant composite (HydroS), and the resulting HydroS is dispersed and/or dissolved in a hydrophilic dispersion medium such as water to prepare S/W. The structure is as shown in Fig. 3.

### 5-3. Solid-in-Oil (S/O)

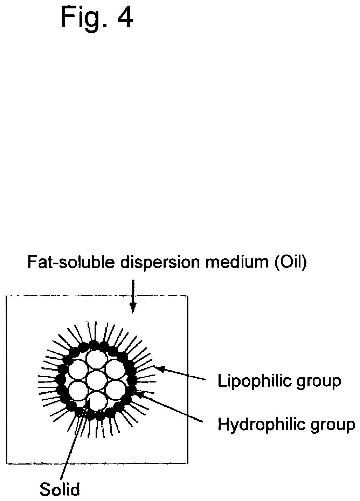

Drugs, and particularly water-soluble drugs, are dissolved in a dissolution tank "a," a highly lipophilic surfactant is dissolved in a dissolution tank "a" or in a dissolution tank "b" in particular, the water-soluble drugs and the surfactant are mixed and emulsified in a emulsification tank "c," followed by removal of water and a solvent, to prepare a drug-surfactant composite (LipoS), and the resulting LipoS is dispersed in a fat-soluble dispersion medium (Oil) that can be used for pharmaceutical, food, or cosmetic products (e.g., vegetable oil) to prepare S/O. The structure is as shown in Fig. 4.

More specifically, S/O can be produced by a method comprising:
(A) a step of dissolving and/or dispersing drugs, and particularly water-soluble drugs, in water or a volatile, hydrophilic solvent, such as alcohol, to obtain a solution and/or dispersion;
(B) a step of liquefying and dissolving a lipophilic surfactant via heating or other means or dissolving and/or dispersing a lipophilic surfactant in an organic solvent to obtain a solution and/or dispersion;
(C) a step of mixing the solution and/or dispersion obtained in step (A) with the solution and/or dispersion obtained in step (B) to obtain an emulsion or dispersion;
(D) a step of removing a solvent and/or moisture from the emulsion or dispersion obtained in step (C); and
(E) a step of dispersing the drug-surfactant composite obtained in step (D) in at least 1 type of fat-soluble solvent selected from among vegetable oils and synthetic oils or fat. Step (A) and

### step (B) may be carried out in any order, and these steps may be carried out simultaneously.

In step (A), drugs are dissolved and dispersed in an aqueous solution via various methods for enhancing solubility. Examples of such methods include a method involving the use of a pH regulator to adjust pH to a level that yields high solubility, addition of a solubilizer, and a method involving heating or heating and pressurization with an inert gas.

In the above-described method, further, low-solubility drugs may be added in the form of fine powder in step (B) to obtain a solution or dispersion. Alternatively, water may further be added thereto to obtain an emulsion. An example of a means for liquefaction of a lipophilic surfactant via heating or other means in step (B) is a method involving heating or heating and pressurization with an inert gas.

### 5-4. Solid-in-Oil-in-Water (S/O/W)

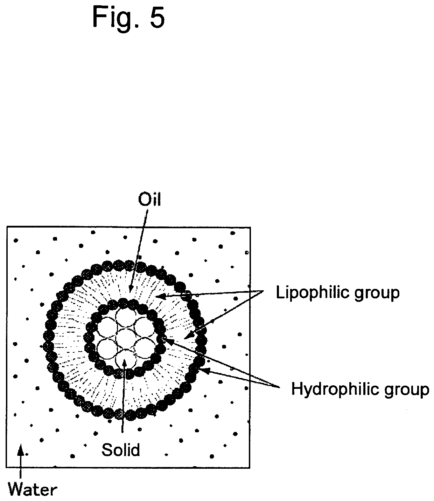

S/O prepared in 5-3. above is dispersed and/or dissolved in a hydrophilic solvent, such as water, to prepare S/O/W. The structure is as shown in Fig. 5.

Also, S/O/W can be produced by a method comprising:
(F) a step of dispersing S/O obtained in step (E) in purified water or a hydrophilic solvent, such as buffer.
5-5. S/W can be produced by a method comprising:
(i) a step of dissolving and/or dispersing drugs, and particularly lipophilic, low-solubility drugs, in an organic solvent to obtain a solution and/or dispersion;
(ii) a step of liquefying and dissolving a hydrophilic surfactant via heating or other means or dissolving and/or dispersing a hydrophilic surfactant in water or a volatile, hydrophilic solvent, such as alcohol, to obtain a solution and/or dispersion;
(iii) a step of adding the solution and/or dispersion in an organic solvent obtained in step (i) to the solution and/or dispersion obtained in step (ii) to obtain an emulsion or dispersion;
(iv) a step of removing a solvent or moisture from the emulsion or dispersion obtained in step (iii) to obtain S; and
(v) a step of dispersing S obtained in step (iv) in purified water or a hydrophilic solvent, such as buffer. Step (i) and step (ii) may be carried out in any order, and these steps may be carried out simultaneously.

In step (ii), drugs may be dissolved and dispersed in an aqueous solution in the form of fine powder to obtain a solution and/or dispersion. Alternatively, an organic solvent may further be added thereto to obtain an emulsion. An example of a means for liquefaction of a hydrophilic surfactant via heating or other means in step (ii) is a method involving heating or heating and pressurization with an inert gas.

In 5-2 to 5-5 above, preferably, steps (A), (B), (C), and at least one of steps (i), (ii), and (iii) are carried out by introducing air or nonflammable gas therein. Preferably, the pressure of the air or inert gas to be introduced is between 1 and 10 atm. More preferably, air or nonflammable gas is introduced into a gas phase in the upper portion of the liquid level at the time of dissolution, dispersion, or emulsification in steps (B), (C), (i), and (iii). Preferably, the pressure of the air or inert gas to be introduced is between 1 and 10 atm.

The nonflammable gas can be at least one type of gas selected from the group consisting of nitrogen, carbonic acid, helium, and argon gases.

Removal of a solvent and/or moisture of step (D) or (iv) can be carried out by means of, for example, vacuum-freeze drying, drying under reduced pressure, microwave drying by a bulking method, freeze-drying and grinding, or spray drying. This step can produce a drug-surfactant composite (S) enclosing drugs in a solid state. According to the present invention, a composite in which the drugs have been subjected to removal of a solvent and/or moisture via such means is defined as "enclosing drugs in a solid state."

Any surfactants that can be used for pharmaceutical, food, and cosmetic products can be used herein. When production of drug-surfactant composites (S) of smaller sizes is intended, for example, HydroS or LipoS is selected in accordance with whether or not drugs are to be dispersed in a hydrophilic solvent or a fat-soluble solvent, and a hydrophilic or lipophilic surfactant is selected.

A variety of types of surfactants can be used herein in accordance with, for example, the intended purpose of products; i.e., whether or not drugs are dissolved or dispersed in a hydrophilic or fat-soluble solvent. The present invention is not limited thereby. Also, two or more types of surfactants can be used in combination.

Any type of surfactant can be used for the drug-surfactant composite of the present invention, provided that such surfactant is used for pharmaceutical, food, and cosmetic products. An ionic or nonionic surfactant may be used. Examples of ionic surfactants include cationic, anionic, and amphoteric surfactants, and one or more surfactants selected therefrom are used.

A lipophilic surfactant is used to generate an S/O emulsion. A hydrophilic surfactant can be added in an amount that is half or less the amount of the lipophilic surfactant. The amount is preferably 10% or less.

An S/O/W preparation obtained by dispersing an S/O oil in an aqueous phase is prepared by adding a hydrophilic surfactant to the aqueous phase. In this case, a lipophilic or hydrophilic surfactant may also be added to the oil phase in an amount that is half or less the amount of the hydrophilic surfactant to be added to the aqueous phase. The amount is preferably 10% or less. Also, a lipophilic surfactant may be added to the aqueous phase. The amount of a lipophilic surfactant to be added to the aqueous phase is half or less the amount of the hydrophilic surfactant, and the amount is preferably 10% or less.

A hydrophilic surfactant is used to generate an S/W emulsion. A lipophilic surfactant in an amount that is half or less the amount of the hydrophilic surfactant can be added. The amount is preferably 10% or less.

Examples of hydrophilic surfactants include anionic, cationic, amphoteric, and nonionic surfactants described below.

Examples of anionic surfactants that can be used include fatty acid soap, naphthenic acid soap, sulfuric acid ester of long-chain alcohol, polyoxyethylene alkylphenyl ether sulfuric acid ester salt, fatty acid monoglyceride sulfuric acid ester, fatty acid monoalkanolamide sulfuric acid ester, alkaline sulfonic acid salt, α-sulfo fatty acid salt, dialkyl sulfosuccinic acid salt, polyoxyethylene octylphenyl ether sulfonic acid salt, alkyl benzene sulfonic acid salt, polyoxyethylene alkylphenol ether phosphoric acid ester salt, polyoxyethylene alkyl ether phosphoric acid ester salt, and sodium lauryl sulfate.

Examples of cationic surfactants that can be used include long-chain primary amine salt, alkyltrimethylammonium salt, dialkyldimethylammonium salt, alkylpyridinium salt, polyoxyethylene alkylamine, and alkyl imidazoline.

Examples of amphoteric surfactants that can be used include N-alkyl β-aminopropionic acid salt and N-alkyl β-iminodipropionic acid salt.

Examples of hydrophilic nonionic surfactants that can be used include an ethylene oxide adduct of a higher alcohol, an ethylene oxide adduct of alkylphenol, an ethylene oxide adduct of fatty acid, an ethylene oxide adduct of polyhydric alcohol-fatty acid ester, an ethylene oxide adduct of higher alkylamine, an ethylene oxide adduct of fatty acid amide, an ethylene oxide adduct of an oil or fat, fatty acid ester of glycerin, fatty acid ester of pentaerythritol, alkyl ether of a polyhydric alcohol, and fatty acid amide of alkanolamines.

Among nonionic surfactants, for example, sorbitol and sorbitan fatty acid esters, polyoxyethylene sorbitan fatty acid ester, polyethylene glycol fatty acid ester, sucrose fatty acid ester, polyoxyethylene castor oil (polyethoxylated castor oil), polyoxyethylene hydrogenated castor oil (polyethoxylated hydrogenated castor oil), polyoxyethylene polypropylene glycol copolymer, fatty acid ester of glycerin, and polyglyceryl fatty acid ester are preferably used.

As polyoxyethylene sorbitan fatty acid ester, for example, polysorbates 20, 40, 60, and 80 are particularly preferable. As polyethylene glycol fatty acid ester, for example, polyethylene glycol monolaurate is particularly preferable. As sucrose fatty acid ester, for example, sucrose palmitic acid esters (e.g., trade name: P-1670, Mitsubishi-Kagaku Foods Corporation), sucrose stearic acid esters (e.g., trade name: S-1670, Mitsubishi-Kagaku Foods Corporation), and sucrose lauric acid esters (e.g., trade name: L-1695, Mitsubishi-Kagaku Foods Corporation) are particularly preferable. As polyoxyethylene castor oil (polyethoxylated castor oil), for example, polyoxyethylene glycerol triricinoleate 35 (Polyoxy 35 Castor Oil; trade name: Cremophor EL or EL-P, BASF Japan Ltd.) is particularly preferable. As polyoxyethylene hydrogenated castor oil (polyethoxylated hydrogenated castor oil), for example, polyoxyethylene hydrogenated castor oil 50 and polyoxyethylene hydrogenated castor oil 60 are particularly preferable. As a polyoxyethylene polyoxypropylene glycol copolymer, for example, polyoxyethylene (160) polyoxypropylene (30) glycol (trade name: Adeka Pluronic F-68, Adeka Corporation) is particularly preferable. As polyglyceryl fatty acid ester, for example, decaglycerol monolaurate (Decaglyn 1-L, Nikko Chemicals Co., Ltd.) is preferable.

Examples of lipophilic surfactant include: sucrose fatty acid esters such as sucrose stearic acid ester, sucrose palmitic acid ester, sucrose oleic acid ester, sucrose lauric acid ester, sucrose behenic acid ester, and sucrose erucic acid ester; sorbitan fatty acid esters such as sorbitan monostearate, sorbitan tristearate, sorbitan monooleate, sorbitan trioleate, and sorbitan sesquioleate; glyceryl fatty acid esters such as glycerol monostearate and glycerol monooleate; and polyglyceryl fatty acid esters such as diglyceryl tetraisostearate, diglyceryl diisostearate, and diglyceryl monoisostearate.

Two or more types thereof may be used.

An oil component or organic solvent that can be used for pharmaceutical, food, or cosmetic products can be used as a fat-soluble solvent to generate S/O or S/O/W of the present invention without particular limitation. Examples thereof include a vegetable oil, an animal oil, a neutral lipid (monosubstituted, disubstituted, or trisubstituted glyceride), a synthetic oil or fat, and a sterol derivative.

Specific examples of vegetable oils include soybean oil, cottonseed oil, rapeseed oil, sesame oil, corn oil, peanut oil, safflower oil, sunflower oil, olive oil, jojoba oil, and perilla oil. Specific examples of animal oils include beef tallow, lard, and fish oil. Specific examples of neutral lipids include triolein, trilinolein, tripalmitin, tristearin, trimyristin, and triarachidonin. Specific examples of synthetic oil or fat include isopropyl myristate, azone, hexane, toluene, chloroform, dichloromethane, ethyl acetate, dimethylacetamide, cyclohexane, acetone, dimethyl sulfoxide, heptane, and pentane. Specific examples of sterol derivatives include cholesteryl oleate, cholesteryl linoleate, cholesteryl myristate, cholesteryl palmitate, and cholesteryl arachidate. These substances may be used in combinations of two or more.

Preferable examples of oil components include triglyceride and a vegetable oil or animal oil comprising triglyceride as an active component. Soybean oil, sesame oil, olive oil, squalane, and squalene are preferable in view of practical use. Highly purified sesame oil or olive oil is particularly preferable.

Contents of drugs and surfactant in the drug-surfactant composite (S) of the present invention vary depending on properties, such as hydrophilicity or lipophilicity. The amount of surfactant relative to drugs is preferably 1:1 to 1:50 by weight, more preferably 1:2 to 1:30, and further preferably 1:5 to 1:20 (i.e., the surfactant:drug ratio).

### 6. Drug formulation as combination preparations

According to the present invention, the drug-surfactant composite (S), S/O, S/W, or S/O/W is prepared in 5. above in accordance with drug properties (i.e., whether or not the drugs are water-soluble drugs, hydrophilic, low-solubility drugs, or lipophilic, low-solubility drugs) to modify the surface properties of drugs relative to a solvent or gel. Thus, substances with different properties can be formulated into a single dosage form. For example, water-soluble drugs can be dispersed in a hydrophilic solvent without modification, but drugs with other properties and water-soluble drugs must be in the form of S/W or S/O/W preparations in order to be dispersed in a hydrophilic solvent. Also, lipophilic, low-solubility drugs can be dispersed in a fat-soluble solvent without modification, but drugs with other properties must be in the form of S/O preparations in order to be dispersed in a fat-soluble solvent.

By modifying drugs in the form of a drug-surfactant composite (S), a plurality of drugs that cannot usually be combined in a single solvent (e.g., drugs that are likely to undergo chemical reactions with each other) are independently contained in a solvent in the form of a drug-surfactant composite. Thus, such drugs can be stably stored without undergoing reaction.

In the present invention, drugs to be dispersed in a solvent are not limited to two types. The present invention is intended to include two or more types of drugs dispersed in a single solvent.

Drugs that are exemplified in 2. above can be used as components of combination preparations.

### 6-1. Combination of water-soluble drugs and low-solubility drugs in a single dosage form

Water-soluble drugs and low-solubility drugs can be combined in a single dosage form in the form of the drug-surfactant composite (S), S/O, S/W, or S/O/W prepared in 5. above.

Examples of water-soluble drugs include VC (vitamin C), cysteine, hydroxyproline, sodium aspartate, glutamine, and arginine. Examples of hydrophilic, low-solubility drugs include branched-chain amino acids (BCAA; i.e., leucine, isoleucine, and valine). An example of a lipophilic low-solubility drug is CoQ10.

Drugs are not limited to a single type, and a plurality of drugs may be used.
6-1-1. Specifically, water-soluble drugs and lipophilic, low-solubility drugs can be dispersed in a hydrophilic solvent (e.g., an aqueous solution) to obtain preparations in the following manner.
   (1) Water-soluble drugs are maintained in that state, lipophilic, low-solubility drugs are modified to the form of HydroS to obtain S/W, and the resulting S/W can be dispersed in a hydrophilic solvent.
   (2) Water-soluble drugs are modified to the form of S/O/W, and lipophilic, low-solubility drugs are modified to the form of HydroS to obtain S/W.

   An aqueous solvent, purified water, buffer, and the like can be used as hydrophilic solvents. Specific examples include an injection solvent, physiologic saline, phosphate buffer, Ringer's solution, acetate buffer, citrate buffer, borate buffer, tartrate buffer, Tris buffer, Macrogol 400, glycerine, propylene glycol, propanol, ethanol, and a mixture of any thereof
6-1-2. When water-soluble drugs and lipophilic, low-solubility drugs are dispersed in a fat-soluble solvent to obtain preparations, lipophilic, low-solubility drugs are dispersed in a fat-soluble solvent in such state, water-soluble drugs are modified to the form of LipoS or S/O as described above, and the resultant can be dispersed in a fat-soluble solvent to obtain preparations.
   Oil components or organic solvents that can be used for pharmaceutical, food, or cosmetic products can be used as fat-soluble solvents without particular limitation. Examples thereof include a vegetable oil, an animal oil, a neutral lipid (monosubstituted, disubstituted, or trisubstituted glyceride), a synthetic oil or fat, and a sterol derivative.
   Specific examples of vegetable oils include soybean oil, cottonseed oil, rapeseed oil, sesame oil, corn oil, peanut oil, safflower oil, sunflower oil, olive oil, jojoba oil, avocado oil, and perilla oil. Specific examples of animal oils include beef tallow, lard, and fish oil. Specific examples of neutral lipids include triolein, trilinolein, tripalmitin, tristearin, trimyristin, and triarachidonin. Specific examples of synthetic oil or fat include isopropyl myristate, azone, hexane, toluene, chloroform, dichloromethane, ethyl acetate, dimethylacetamide, cyclohexane, acetone, dimethyl sulfoxide, heptane, and pentane. Specific examples of sterol derivatives include cholesteryl oleate, cholesteryl linoleate, cholesteryl myristate, cholesteryl palmitate, and cholesteryl arachidate. These substances may be used in combinations of two or more.
   Preferable examples of oil components include triglyceride and a vegetable oil or animal oil comprising triglyceride as an active component. Soybean oil, sesame oil, olive oil, squalane, and squalene are preferable in view of practical use. Highly purified sesame oil or olive oil is particularly preferable.
   Further, the dispersion of drugs in a fat-soluble solvent can fill a soft or hard capsule to result in a preparation, and such soft or hard capsule can be further packaged in a blister pack.
6-1-3. When water-soluble drugs and hydrophilic, low-solubility drugs are dissolved in a fat-soluble solvent, both drugs are dispersed in an oil phase in an LipoS form to obtain S/O preparations, and the resulting S/O preparations can then be dispersed in a fat-soluble solvent.
   Fat-soluble solvents described in 6-1-2 above can be used.
6-1-4. When water-soluble drugs and hydrophilic, low-solubility drugs are dispersed in a hydrophilic solvent, hydrophilic, low-solubility drugs are dispersed in an oil phase in an LipoS form to obtain S/O preparations, the resulting S/O preparations are dispersed in an aqueous phase to obtain S/O/W preparations, and the resulting S/O/W preparations can then be dispersed in a hydrophilic solvent.
   Hydrophilic solvents described in 6-1-1 above can be used.
6-1-5. When water-soluble drugs and low-solubility drugs are used in a solid or gel state, other solid or gel components can be mixed with HydroS and/or LipoS.
   Examples of other solid or gel components include carboxymethylcellulose sodium, sodium alginate, carrageenan, pectin, agar, gellan gum, glucomannan, polyvinyl pyrrolidone, carboxyvinyl polymer, tragacanth, amide acrylate, and polyvinyl alcohol.
   Hydrophilic, low-solubility drugs may be modified to an LipoS form and then dispersed in aqueous gel via the effects of a gel base material.

### 6-2. Combination of a plurality of drugs that cannot exist together due to chemical reactions in a solvent

The term "chemical reaction" used herein refers to any chemical reaction that affects drugs. Examples thereof include, but are not limited to, an oxidation-reduction reaction, a chelate reaction, an anion-cation reaction, an addition reaction, and an amino carbonyl reaction.

### 6-2-1. Drugs that cause oxidation-reduction reaction

Drugs having oxidizing properties and drugs having reducing properties cannot be combined in a single dosage form. However, the technique of the present invention is capable of realizing such combination.

Examples of drugs having oxidizing properties include miconazole nitrate, isosorbide dinitrate, and naphazoline nitrate. Examples of drugs having reducing properties include vitamin C, cysteine, acetylcysteine, cinnamic aldehyde, vitamin E, beta carotene, folic acid, uric acid, ubiquinone, ethyl icosapentate, glutathione, and sodium sulfite.

### 6-2-2. Anionic drug and cationic drug

Acidic drugs (anionic drugs) and basic drugs (cationic drugs) cannot be combined in a single dosage form. However, the technique of the present invention is capable of realizing such combination.

Examples of anionic drugs include nucleotide drugs such as adenosine triphosphate, RNA, and DNA, valproic acid, probenecid, penicillins, indomethacin, felbinac, warfarin, salicylic acid, aspirin, phenobarbital, sodium azulene sulfonate, alprostadil, amlexanox, and glycyrrhetic acid. Examples of cationic drugs include aminoglycoside antibiotics such as doxorubicin, olopatadine, dopamine, benidipine, and micronomicin, domperidone, vinorelbine, tropisetron, cimetidine, procainamide, tetracycline, imipramine, amphetamine, and meperidine.

### 6-2-3. Chelating drugs

Drugs that cause complexation (chelation) and drugs that cause metal-ion exchange reactions cannot be combined in a single dosage form. However, the technique of the present invention is capable of realizing such combination.

Examples of combinations of such drugs include a combination of aluminum hydroxide with a new quinolone drug and that of a metal chelator (e.g., edetate calcium disodium, dimercaprol, or deferoxamine) with a metal ion-containing drug (e.g., cyanocobalamin, methylcobalamin, a cisplatin type antitumor platinum complex, or aceglutamide aluminium). When a cisplatin type antitumor platinum complex (e.g., cisplatin, carboplatin, nedaplatin, or oxaliplatin) is mixed with a solution containing Na ions or Ca ions, such as physiological saline or a Ringer's solution, further, ligand exchange takes place. However, such drugs can be combined via preparation of a drug-surfactant composite according to the present invention.

### 6-2-4. Amino carbonyl reaction

Amino acids or amino-containing drugs cannot be combined with saccharides or aldehyde-containing drugs because amino carbonyl reactions take place. However, the technique of the present invention is capable of realizing such combination.

Examples of amino acids or amino-containing drugs include various amino acids, interferon, human growth hormone, granulocyte growth factor, insulin, salmon calcitonin, other protein- and peptide-based drugs, dopamine, acetylsulfamethoxazole, allantoin, norephedrine, and aminophylline. Examples of saccharides and aldehyde-containing drugs include glucose, lactose, sucrose, aceglatone, tribenoside, and vidarabine.

### 6-2-5. Pharmaceutical preparation

A plurality of drugs that cause chemical reactions are classified as water-soluble drugs, hydrophilic, low-solubility drugs, or lipophilic, low-solubility drugs.

When the plurality of drugs are water-soluble drugs, (i) drug-surfactant composites (LipoS) of each drug are prepared and dispersed in an oil phase (Oil) to prepare a Solid-in-Oil (S/O) preparation, and the resulting S/O preparation is dispersed in a fat-soluble solvent to obtain a pharmaceutical preparation; or (ii) drug-surfactant composites (LipoS) of each drug are dispersed in an oil phase (Oil) to prepare a Solid-in-Oil (S/O) preparation, the resulting S/O preparation is further dispersed in an aqueous phase to prepare a Solid-in-Oil-in-Water (S/O/W) preparation, and the resulting S/O/W preparation is dispersed in a hydrophilic solvent to obtain a pharmaceutical preparation.

When the plurality of drugs are lipophilic, low-solubility drugs, drug-surfactant composites (HydroS) of each drug are prepared and dispersed in an aqueous phase (Water) to prepare a Solid-in-Water (S/W) preparation, and the resulting S/W preparation is dispersed in a hydrophilic solvent to obtain a pharmaceutical preparation.

When the plurality of drugs are hydrophilic, low-solubility drugs, drug-surfactant composites (LipoS) of each drug are dispersed in an oil phase (Oil) to prepare a Solid-in-Oil (S/O) preparation, the resulting S/O preparation is further dispersed in an aqueous phase to prepare a Solid-in-Oil-in-Water (S/O/W) preparation, and the resulting S/O/W preparation is dispersed in a hydrophilic solvent to obtain a pharmaceutical preparation.

When the plurality of drugs are hydrophilic, low-solubility drugs and are dispersed in aqueous gel, drug-surfactant composites (Solid) of each drug are dispersed in an aqueous phase (Water) to prepare a Solid-in-Water (S/W) preparation, and the resulting S/W preparation is dispersed in aqueous gel to obtain a pharmaceutical preparation.

Drug-surfactant composites can be prepared as described in the "5. Drug-surfactant composite" section above.

Hydrophilic solvents and fat-soluble solvents described in 6-1-1 and 6-1-2 above can be used.

### 6-3. Additives and dosage forms

When the pharmaceutical preparation (i.e., the drug composition) comprising drugs having different physical properties combined in a single dosage form of the present invention is a liquid or slurry, such liquid or slurry may be absorbed by additives such as lactose, silica gel, crystalline cellulose, or starch that can be used for pharmaceutical, food, or cosmetic products. Thus, such liquid or slurry can be modified to a powder form.

Additives such as stabilizers that are used for the pharmaceutical preparation (i.e., the drug composition) comprising drugs having different physical properties combined in a single dosage form of the present invention can be selected from among additives used for pharmaceutical, food, or cosmetic products. For example, proteins such as bovine serum albumin, egg albumin, or milk casein, saccharides such as maltose, sucrose, or trehalose, or polysaccharides such as heparin, chitosan, alginic acid, polyacrylic acid, polymethacrylic acid, or carboxymethylcellulose can be freely selected in accordance with the intended purpose while taking drug physical properties or compatibility into consideration. The present invention is not limited to the aforementioned examples.

Examples of additives used for stabilizing S/O, S/W, and S/O/W preparations include protective colloids and thickeners. Examples of protective colloids include amino acids, proteins, saccharides, and polysaccharides. Examples of thickeners include water-soluble polysaccharides such as carboxymethylcellulose sodium, sodium alginate, sodium hyaluronate, and dextran. Examples of thickeners used for an oil phase include various fatty acids and oils and fats.

Examples of preservatives include alkyl parabens, such as methylparaben and propylparaben.

Examples of soothing agents include benzyl alcohol, xylocaine hydrochloride, and chlorobutanol.

Examples of pH regulators include hydrochloric acid, acetic acid, sodium hydroxide, and various buffers.

### 6-4. Application of drug composition

The pharmaceutical preparation (i.e., the drug composition) comprising drugs having different physical properties combined in a single dosage form of the present invention can be used for a pharmaceutical product alone or in combination with other components. Examples of the pharmaceutical product include an injection preparation, an internal agent, an external preparation, eye drops, and a dental drug. Further examples include injection preparations, eye drops, internal agents, such as powders, granules, tablets, capsules, liquid preparations, and jellies, adhesive skin patches, such as poultices and tapes, external preparations, such as ointments, creams, lotions, liquid preparations, and sprays, and dental drugs, which are obtained via solubilization of low-solubility drugs. Such dosage forms may be prepared in accordance with a conventional technique, and the present invention is not limited by such technique.

The pharmaceutical preparation (i.e., the drug composition) comprising drugs having different physical properties combined in a single dosage form of the present invention can be used for a food product alone or in combination with other components. Examples of food products include common food, food additives, milk, milk beverages, dairy products such as cheese or yogurt, processed milk, popular beverages, seasonings, spices, refreshing beverages, processed beverages, food with health-promoting benefits, health-promoting food referred to as food for specified health use or food with nutrient function claims, and food for special use. It should be noted that the present invention is not limited thereto.

Further, examples of cosmetic products that use the drug composition of the present invention include, but are not limited to, foundations, facial masks, creams, emulsions, skin lotions, beauty essence, cleansing products, lipsticks, lipliner, gloss, eyelash liner, eye shadow, eye liner, eyebrow products, and cheek colorants.

### 7. Masking or deodorization of drugs or stabilization of drug storage with drug-surfactant composite

The present invention includes a method for enhancing the stability of a drug lacking stability in a solvent or a method for masking or deodorizing with the use of the drug-surfactant composite described in 5. above of a drug that lacks stability in a solvent or a drug that exhibits odor or an unpleasant taste.

Examples of relevant drugs include those exemplified in 2 above. In the method of masking or deodorization, in particular, drugs exhibiting strong odors such as cysteine or drugs exhibiting unpleasant tastes such as bitterness (e.g., branched-chain amino acids (i.e., BCAA)) are particularly preferable. Examples of drugs that are used for stabilization include drugs that are immediately dissolved in a solvent and drugs that are oxidized or reduced. Specific examples include vitamin C and derivatives thereof.

The hydrophilic or fat-soluble solvents described in 6 above can be used.

Hereafter, Production Examples and Examples are described, although the present invention is not limited thereto.

### [Production Example 1] Production of drug-surfactant composite (S)

### (Solubilization of water-soluble drugs in fat; hereafter referred to as "LipoS")

Sucrose fatty acid ester (ER-290, 7.9 g) was completely dissolved in 75 g of hexane. Separately, 0.1 g of citric acid and 2 g of magnesium ascorbyl phosphate (i.e., a vitamin C derivative; hereafter abbreviated as "VC·PMg") were added thereto and completely dissolved in 50 g of purified water. This aqueous solution was added to a hexane solution comprising the sucrose fatty acid ester (ER-290) dissolved therein, and the resultant was emulsified with the use of a homogenizer at 20,000 rpm for 5 minutes. Thereafter, a hexane phase was removed with the use of an evaporator. After the solvent was removed, the resultant was frozen at -45°C for lyophilization, and the solvent was completely removed. Thus, the VC·PMg-surfactant composite (VC·PMg-LipoS) was obtained.

### [Production Example 2] Production of drug-surfactant composite (S)

### (Hydrophilization of low-solubility drugs, hereafter referred to as "HydroS")

Sodium lauryl sulfate (SLS, 120 g) was completely dissolved in 1,200 g of purified water, and 120 g of hexane comprising 40 g of ubiquinone (hereafter referred to as "CoQ10") dissolved therein was added thereto during agitation at 1,500 rpm using a lamond stirrer. The resulting solution was subjected to membrane emulsification with the use of a 0.1-µm lipophilic membrane, and the solution was then frozen at -45°C for lyophilization. Thus, the CoQ10-surfactant composite (CoQ10-HydroS) was obtained.

### [Production Example 3] Production of drug-surfactant composite (S)

### (Masking of low-solubility drugs; BCAA/LipoS)

Three types of lipophilic amino acids; i.e., 1.907 g of leucine, 0.952 g of isoleucine, and 1.144 g of valine (branched-chain amino acids; hereafter abbreviated as "BCAA"), were dissolved in 100 g of purified water at 60°C, the resulting solution was added to a solution of 12 g of sucrose fatty acid ester (ER-290) completely dissolved in 150 g of n-hexane, and the resultant was subjected to high-speed agitation (20,000 rpm) with the use of a homogenizer to prepare a W/O emulsion solution. The resulting emulsion solution was subjected to lyophilization overnight to obtain a BCAA-surfactant composite.

### [Production Example 4] Production of Solid-in-Water (S/W)

CoQ10-HydroS (5 g) obtained via surface modification of CoQ10 low-solubility drugs into hydrophilic surfaces in Production Example 2 was completely dissolved in 45 g of purified water to obtain S/W CoQ10.

### [Production Example 5] Production of Solid-in-Oil (S/O)

0.5 g of VC·PMg-LipoS obtained in Production Example 1 was added to and dissolved in Isopropyl myristate (9.5 g) to obtain S/O VC·PMg.

### [Production Example 6] Production of Solid-in-Oil-in-Water (S/O/W)

S/O VC·PMg (1 g) obtained in Production Example 5 was dispersed and dissolved in 9 g of an aqueous solution of 0.1 % polyoxyethylene hydrogenated castor oil 60 to obtain S/O/W VC·PMg.

### [Production Example 7] Production of CoQ10-HydroS tablet

Sodium L-aspartate (900 g), 900 g of L-glutamine, and 900 g of L-arginine were mixed with 1,200 g of CoQ10-HydroS obtained in Production Example 2,312 g of Carplex was introduced thereinto, and granule size was adjusted with the aid of a power mill (20-mesh). Crystalline cellulose (1,560 g) and 9,672 g of Perfiller 102 were introduced and mixed therewith, 156 g of sugar ester was then added, and the mixture was subjected to tablet making to obtain CoQ10-HydroS tablets.

### [Production Example 8] Production of VC·PMg-LipoS cream

Plastibase (98 g), 32 g of behenyl alcohol, 16 g of silicone oil, and 20 g of squalane were fractionated, and these substances were heated at 90°C and completely dissolved during agitation at 1,500 rpm using a lamond stirrer. After heating, the agitation speed was adjusted to 1,000 rpm and the product was cooled to 50°C. A solution of 10 g of VC·PMg-LipoS dissolved in 30 g of coconut oil was added thereto, 4 g of talc and 0.2 g of sodium pyrosulfife were further added, and the mixture was agitated at 2,500 rpm for 10 minutes. The resultant was cooled to 40°C and an aluminum tube was filled therewith to obtain a VC·PMg-LipoS cream (S/O).

### [Production Example 9] Production of VC·PMg-LipoS oil

1 g of VC·PMg-LipoS obtained in Production Example 1 was added to and dissolved in Coconut oil (9 g) with agitation, and the resulting solution was fractionated to vanity glass cases for a given purpose to obtain VC·PMg-LipoS oil (S/O).

### [Production Example 10] Production of BCAA-LipoS jelly

Purified water (64 g) was added to a container, and 0.1 g of citric acid was added thereto and completely dissolved therein with agitation. Sorbitol (10 g) and 16 g of BCAA-LipoS obtained in Production Example 3 were introduced thereinto, the mixture was agitated at 1,500 rpm for 5 minutes, and the resultant was heated to 60°C.

Separately, 0.54 g of carrageenan, 0.18 g of xanthan gum, and 0.09 g of purified locust bean gum were uniformly dispersed in 9 g of concentrated glycerine, the entire amount of the resulting dispersion was introduced, the agitation speed was increased to 5,000 rpm, and the temperature was raised to 85°C. Thereafter, the agitation speed was reduced to 3,000 rpm, 0.09 g of propylparaben was introduced thereinto, and the resultant was heat-sterilized for 30 minutes. The temperature was reduced to 60°C while maintaining the agitation speed at 3,000 rpm to obtain a BCAA-LipoS jelly (S/W).

### [Production Example 11] Production of BCAA-LipoS oil

16 g of BCAA-LipoS obtained in Production Example 3 was added to and dissolved in medium-chain triglyceride (34 g) with agitation, and the resultant was packaged with the use of a liquid and viscous filling machine to obtain BCAA-LipoS oil (S/O).

### [Production Example 12] Production of drug-surfactant composite (S)

### (Solubilization of water-soluble drugs in fat; Production Example of LipoS)

Sucrose fatty acid ester (ER-290, 18 g) was completely dissolved in 75 g of hexane. Separately, 2 g of VC·PMg was added thereto and completely dissolved in 50 g of purified water. This aqueous solution was added to a hexane solution comprising the sucrose fatty acid ester (ER-290) dissolved therein, and the resultant was emulsified with the use of a homogenizer at 20,000 rpm for 5 minutes. Thereafter, a hexane phase was removed with the use of an evaporator. After the solvent was removed, the resultant was frozen at -45°C for lyophilization, and the solvent was completely removed. Thus, a VC·PMg-surfactant composite (VC·PMg-LipoS) was obtained.

### [Production Example 13]

### (Solubilization of water-soluble drugs in fat; Production Example of LipoS oil)

Medium-chain triglyceride (30 g) was added to 90 g of sucrose fatty acid ester (ER-290), and the resultant was agitated until a homogeneous solution was obtained. This solution was designated as a surfactant-oil solution. L-cysteine (10 g) was dissolved in 70 g of purified water in another vessel, this aqueous solution was added to the surfactant-oil solution, and the resultant was emulsified with the use of a homogenizer at 20,000 rpm for 5 minutes. Thereafter, the resultant was frozen at -45°C for lyophilization, and moisture was completely removed. Thus, an S/O cysteine-surfactant composite (LipoS) was obtained.

### [Production Example 14]

### (Hydrophilization of lipophilic, low-solubility drugs, Production Example of HydroS)

Ubiquinone (CoQ10, 2 g) was completely dissolved in 75 g of hexane. Separately, 18 g of SLS was added thereto and completely dissolved in 50 g of purified water. This aqueous solution was added to the solution of CoQ10 dissolved in hexane, and the resultant was emulsified with the use of a homogenizer at 20,000 rpm for 5 minutes. Thereafter, a hexane phase was removed with the use of an evaporator. After the solvent was removed, the resultant was frozen at -45°C for lyophilization, and the solvent was completely removed. Thus, a CoQ10-surfactant composite (CoQ10-HydroS) was obtained.

### [Production Example 15]

### (Solubilization of water-soluble drugs in fat; Production Example of LipoS)

Sucrose fatty acid ester (ER-290, 45 g) was completely dissolved in 300 g of hexane. Separately, 5 g of hydroxyproline (hereafter abbreviated as "HP") was added thereto and completely dissolved in 30 g of purified water. This aqueous solution was added to a hexane solution comprising the sucrose fatty acid ester (ER-290) dissolved therein, and the resultant was emulsified with the use of a homogenizer at 20,000 rpm for 5 minutes. Thereafter, a hexane phase was removed with the use of an evaporator. After the solvent was removed, the resultant was frozen at -45°C for lyophilization, and the solvent was completely removed. Thus, an HP-surfactant composite (HP-LipoS) was obtained.

### [Production Example 16] Production of Solid-in-Oil (S/O)

Perilla oil (9.5 g) was added to dissolve 0.5 g of VC·PMg-LipoS obtained in Production Example 1 to obtain S/O VC·PMg.

### [Production Example 17] Production of Solid-in-Oil (S/O)

Perilla oil (9.5 g) was added to dissolve 0.5 g of HP-LipoS obtained in Production Example 15 to obtain S/O HP.

### [Production Example 18] Production of Solid-in-Oil (S/O)

Perilla oil (9 g) was added to dissolve 0.5 g of VC·PMg-LipoS obtained in Production Example 1 and 0.5 g of HP-LipoS obtained in Production Example 15 to obtain a mixed solution of S/O VC·PMg and S/O HP.

### Example 1

### (Stabilization of drug by S/O preparation)

Long-term stability (2-year) was inspected at room temerature using S/O VC·PMg obtained in Production Example 5. A non-S/O VC·PMg solution was used as a positive control, and a dispersion medium used for S/O preparation was used as a negative control.

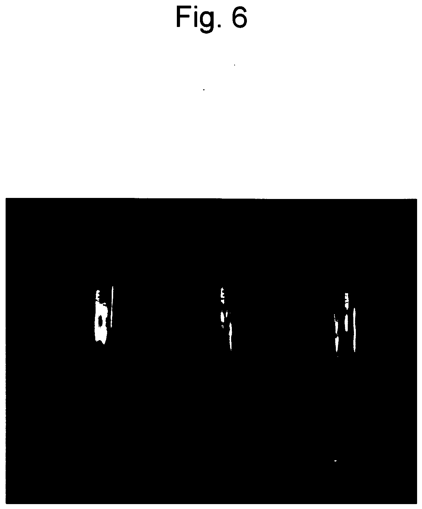

The photograph shown in Fig. 6 was stored at room temperature for 2 years. The photograph shows, from left to right, S/O VC·PMg, a VC·PMg solution (a positive control), and a dispersion medium (O) for S/O (a negative control). The VC·PMg solution was browned with the elapse of time, and S/O VC·PMg was not browned at all in the state of a dispersion medium. This indicates that S/O VC·PMg is very stable.

### Example 2

### (Improvement in drug absorption through skin by S/O preparation)

Improvement in absorption of S/O VC·PMg obtained in Production Example 5 through the skin was inspected. As a positive control, an aqueous solution of trisodium ascorbyl palmitate phosphate (i.e., a vitamin C derivative; hereafter abbreviated as "APPS"), in which the same amount of vitamin C as the sample (i.e., 10 mg/ml) was contained, was used. As a negative control, an aqueous solution of non-S/O VC · PMg, in which the same amount of VC·PMg as the sample was contained, was used.

The samples were applied to the abdominal region of rats under ether anesthesia in amounts of 50 µl per 1 cm². The dermal tissue was peeled from the applied region four hours later, weighed, and subjected to hydrazine colorimetry to quantify the vitamin C content.

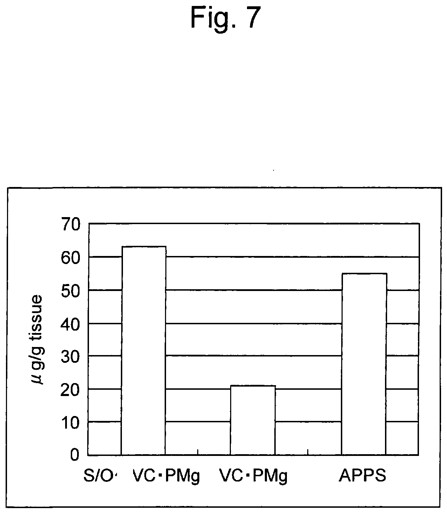

In Fig. 7, the vertical axis represents vitamin C content (µg) relative to the weight of rat dermal tissue (g), and the horizontal axis represents, from left to right, a group to which S/O VC·PMg was applied, a group to which VC·PMg was applied (a negative control group), and a group to which APPS was applied (a positive control group).

The group to which S/O VC·PMg was applied exhibited a significant increase in vitamin C content in the dermal tissue in comparison with the group to which only VC·PMg was applied. Such increase was equivalent to or greater than the increase attained by APPS, which was another vitamin C derivative. This indicates that preparation of drugs in the form of an S/O preparation significantly improves vitamin C absorption through the skin. Since APPS is poor in stability, it is apparent that S/O VC·PMg is superior to APPS because of its satisfactory stability.

### Example 3

### (Masking of bitterness or acridness of nutritional factor with drug-surfactant composite)

Healthy volunteers (8 individuals) were subjected to a functional test using the branched-chain amino acid obtained in Production Example 10.

The functional test was carried out with the use of two types of samples: 1) S branched-chain amino acid; and 2) a mixture of the same amounts of a branched-chain amino acid and a surfactant. In order to avoid the influence of odor that is characteristic of the branched-chain amino acid or influence on taste determination, lemon oil was added to the samples in amounts of 0.01 % by weight.

In the functional test, samples were numbered at random, so that the subjects would not be able to deduce the nature of the samples based on regularity of sample numbers, samples (0.5 g each) were supplied to the subjects, and the subjects were asked to rank the samples.

The functional test was carried out twice, and the results are as shown in Table 1. The results demonstrate that preparation of drugs in the form of an S preparation significantly improves unpleasant texture, such as in terms of bitterness and acridness.

**Table 1: Effects of masking bitterness and acridness of nutritional factor with drug-surfactant composite**

| Bittemess/acridness | A | B | C | D | E | F | G | H |
|---|---|---|---|---|---|---|---|---|
| 1) First | None | Weak | Weak | None | Weak | None | None | Weak |
| 2) First | Strong | Strong | Strong | Strong | Strong | Strong | Strong | Strong |
| 1) Second | None | Weak | Weak | Weak | Weak | None | None | Weak |
| 2) Second | Strong | Strong | Strong | Strong | Strong | Strong | Strong | Strong |

| | | | | | | | | |
|---|---|---|---|---|---|---|---|---|
| Functional test with 8 healthy volunteers (A to H) (Bitterness and acridness levels are scored as none, weak, mild, and strong.) 1) S branched-chain amino acid (A leucine-isoleucine-valine mixture is prepared in the form of an S jelly preparation with ER-290) 2) Two types of samples of same amounts of mixtures of branched-chain amino acids and surfactants (Physical mixtures of leucine, isoleucine, valine, and ER-290) | | | | | | | | |

### Example 4

### (Combination of water-soluble drugs and lipophilic, low-solubility drugs in single dosage form via solubilization of water-soluble drugs in fat)

The VC·PMg-surfactant composite (VC·PMg-LipoS, 5g) obtained in Production Example 12 and 0.5 g of vitamin E (VE) were dispersed in 1 kg of olive oil with the use of an agitation blade, and a clear homogeneous solution was obtained. This homogeneous solution was subjected to plastic filling with the use of a rotary soft capsule filling machine in constant amounts of 0.4 g, and soft capsules containing VC·PMg and VE with high content uniformity were obtained. Soft capsules could be produced in a twist-off form as well as in a general oval form. Similarly, constant amounts of 0.1 g were subjected to plastic filling with the use of a seamless soft capsule filling machine to obtain soft capsules containing VC·PMg and VE with high content uniformity. In contrast, a clear solution was not obtained when 0.5 g of VC·PMg and 0.5 g of VE were directly dispersed in 1 kg of olive oil containing 4.5 g of sucrose fatty acid ester (ER-290). In this case, VC·PMg remained suspended in the solution. When such solution was allowed to stand, a precipitate was observed, and soft capsules could not be formed. The formed soft capsules were packaged in easy-peel blister packs composed of a plastic film and an aluminum film. Thus, products that allow soft capsules with a low degree of hardness to be easily removed and are clean and easy to carry were obtained. Soft capsules were also packaged in press-through blister packs.

Thus, VC·PMg, which is a water-soluble nutritional factor, was prepared in the form of a VC·PMg-surfactant composite (VC·PMg-LipoS). This enabled homogeneous dispersion or dissolution of VC·PMg in vegetable oil with VE, which is a lipophilic, low-solubility nutritional factor.

### Example 5

### (Example of S/W comprising CoQ10-HydroS microdispersed/dissolved in aqueous solution for electrode gel containing drugs for iontophoresis)

Carbopol (Carbopol 940, manufactured by B. F. Goodrich, 0.2 g) was dissolved in 0.3% citrate buffer (pH 6). The pH level of this solution was adjusted at 6 with sodium hydroxide to bring the total amount of the solution to 50 g, and a solution of 0.4% Carbopol (hereafter referred to as an "aqueous 0.4% Carbopol solution") was obtained. The CoQ10-surfactant composite (CoQ10-HydroS, 5 g) obtained in Production Example 14 was dispersed in 50 g of an aqueous 0.4% Carbopol solution with the use of an agitation blade, and a clear homogeneous solution was obtained (hereafter referred to as an "aqueous 0.4% Carbopol solution containing CoQ10-HydroS").

With the use of an iontophoresis apparatus, a positive electrode of an electrostimulator was connected to a carbon electrode, and a negative electrode thereof was connected to a silver chloride electrode. An aqueous 0.4% Carbopol solution (2 ml) was introduced into a cell at the positive electrode, 2 ml of an aqueous 0.4% Carbopol solution containing CoQ10-HydroS was introduced into a cell at the negative electrode, and a 0.2 mA current was applied for 30 minutes.

In contrast, a clear solution was not obtained when 0.5 g of CoQ10 was directly dispersed in 50 g of an aqueous 0.4% Carbopol solution containing 4.5 g of SLS. In this case, CoQ10 remained suspended in the solution. When such solution was allowed to stand, a precipitate was observed, and an aqueous solution for an electrode gel containing a nutritional factor for iontophoresis was not prepared. Thus, CoQ10, which is a low-solubility, nutritional factor, was prepared in the form of a CoQ10-surfactant composite (CoQ10-HydroS), and an aqueous solution for an electrode gel containing a nutritional factor for iontophoresis homogeneously dispersed/dissolved in water was obtained.

### Example 6

### (Masking of odor via preparation of cysteine-LipoS oil preparation)

Healthy volunteers (8 individuals) were subjected to a functional test regarding odor of the cysteine-LipoS oil obtained in Production Example 13. As a result, all 8 subjects determined that characteristic odor of cysteine was reduced.

### Example 7

### (Increase in collagen amount via percutaneous administration of S/O HP and S/O VC·PMg)

Male SD rats (27 rats, body weight: 250 g) were divided into the 5 groups shown below and subjected to a percutaneous administration test. Control group 1): a solution of 1% VC·PMg and 1% HP dispersed in perilla oil was administered. Test group 2): an S/O VC·PMg solution containing 1% VC·PMg obtained in Production Example 16 was administered. Test group 3): an S/O HP solution containing 1% HP obtained in Production Example 17 was administered. Test group 4): a solution containing 1% S/O VC·PMg + 1% S/O HP obtained in Production Example 18 was administered. These 4 groups each consisted of 6 rats. Negative control group 5): perilla oil was administered, and 3 rats were employed. Testing was carried out.

The backs of rats were shaved under ether anesthesia, the back skin was cut to a length of 5 cm to the fascia with scissors, the incision sites were closed with the use of Vista t (R) (skin staplers) to obtain skin wounds, 100 µl of the sample was applied to the surfaces of wounds once a day, and such application was continued for 17 days.

In the control group to which 1% VC·PMg + 1% HP had been administered, the collagen content in the dermal tissue did not increase; however, no significant increase was observed even though 1%S/O VC·PMg +1%S/O HP had been percutaneously administered. Such increase was not observed via percutaneous administration of S/O VC·PMg alone to Test group 2) or percutaneous administration of S/O HP alone to Test group 3). Accordingly, it is apparent that simultaneous percutaneous administration of VC·PMg and HP in the form of an S/O preparation results in an increase in collagen content.

### Industrial Applicability

The present invention is intended to provide a product comprising a drug-surfactant composite, such as a pharmaceutical preparation or paramedical product, which can comprise two or more types of drugs having different physical properties or drug-surfactant composites in a single dispersion medium by preparing a drug-surfactant composite comprising drugs enclosed by a surfactant. It is intended to homogenize physical properties of drugs, microdisperse drugs in a hydrophilic or fat-soluble solvent, stabilize drugs, enhance absorbability, and mask bitterness, acridness, or odor.

Different physical properties of drugs can be homogenized, the resulting drugs are stable and excellent in absorbability, and problems such as bitterness, acridness, or odor can be improved.

By converting drug surfaces to hydrophilic and fat-soluble surfaces to homogenize surface properties of a plurality of drugs according to need, water-soluble or fat-soluble pharmaceutical preparations can be produced. By rendering the surfaces hydrophilic, drugs can be ionized, and drugs also can be solubilized in a water-soluble solvent.

Since two or more types of drugs having different physical properties can be stably combined in one dispersion medium, uneven drug contents resulting from suspension of powders, precipitation or phase separation resulting from heating, and aging variation over time due to storage can be prevented. In addition, this technique is capable of preventing direct contact of drugs, which causes changes in drug compositions, and thus is capable of inhibiting changes in drug compositions.

Examples of changes in drug compositions include an addition reaction between vitamin C and sodium pyrosulfife, an amino carbonyl reaction between an amino acid or an amino-group-containing drug and a saccharide or aldehyde-group-containing drug, a precipitation reaction between a compound having a sulfuric acid group and a drug having an amino group, and changes in drug composition of terbinafine hydrochloride and benzalkonium chloride. Other examples include changes in drug compositions of polyethylene glycol and phenobarbital and of polyethylene glycol and a vinca alkaloid drug, changes in drug compositions of dibutyl hydroxytoluene and an amine-containing compound, Schiff base formation involving aminophylline and glucose, the furfural reaction involving tetracycline and alkaline earth, racemization of epinephrine by a sulfurous acid group, acid amide formation involving benzocaine and citric acid, formation of a gentamicin-carbenicillin composite, aminolysis involving aspirin and phenylephrine, and epimerization involving multivitamin preparations. Development of techniques that overcome such drawbacks has been awaited. Thus, the method of the present invention comprising coating a surface (or surfaces) of one component (or both components) with a surfactant to prepare a drug-surfactant composite has enabled inhibition of changes in drug composition.

Examples of combinations of low-solubility drugs and water-soluble drugs, low-solubility drugs and other low-solubility drugs, and water-soluble drugs and other water-soluble drugs into a single dosage form for the purpose of enhancing the effects of drugs or inhibiting side effects include a combination of eicosapentaenoic acid and Lipitor (i.e., therapeutic agents for hyperlipidemia), a combination of vitamin K2 and lansoprazole (i.e., a proton pump inhibitor), a combination of a calcium antagonist and an ACE inhibitor, a combination of haloperidol decanoate and haloperidol, a combination of vitamin E and vitamin C, a combination of vitamin A and vitamin C, a combination of ascorbyl stearate and ascorbic acid or magnesium ascorbyl phosphate, a combination of coenzyme Q10 and magnesium ascorbyl phosphate, and a combination of indomethacin and diclofenac sodium. When such components were combined as liquid agents according to conventional techniques, however, one of the drugs was precipitated, suspended, aggregated, or underwent changes in drug composition with pharmaceutical additives when either a water-soluble or fat-soluble solvent was used. Thus, it was impossible to develop pharmaceutical preparations exhibiting high content uniformity. According to the method of the present invention, a surface (or surfaces) of one component (or both components) was (or were) coated with a surfactant to prepare a drug-surfactant composite, and such components were combined into a single dose.

The present invention provides a drug-surfactant composite Solid (S) and dispersions thereof; i.e., a Solid-in-Water (S/W) dispersion, a Solid-in-Oil (S/O) dispersion, and a Solid-in-Oil-in-Water (S/O/W) dispersion, comprising drugs used for food or cosmetic products as well as for pharmaceutical products enclosed by surfactants. This can arbitrarily changes physical properties of drugs, which consequently enables dispersion or dissolution of water-soluble drugs and low-solubility drugs in a single solution, stabilization of drugs, enhancement of drug absorption into living organisms, and masking of the bitterness or acridness of drugs. Thus, a variety of products, such as oil injection preparations, percutaneous agents, tablets, or hard capsules can be prepared, in addition to soft capsules or oral jelly preparations.

The method of the present invention makes low-solubility drugs soluble in water, which enables homogeneous dispersion or dissolution of low-solubility drugs and water-soluble drugs in water. Such liquid can be prepared in the form of oral jelly preparations, injection preparations, percutaneous agents, tablets, or hard capsules.

In particular, HydroS comprising low-solubility drugs enclosed by a hydrophilic surfactant can be homogeneously dispersed or dissolved in an aqueous solution for iontophoresis electrode (S/W). Thus, percutaneous absorption can be expected via iontophoresis. Such technique is also applicable to solutions of nutritional factors used for an ion introduction technique, which is a similar method for accelerating percutaneous absorption, sonophoresis, which is a method for accelerating percutaneous absorption with ultrasound, and electroporation.

## Claims

1. A pharmaceutical preparation comprising one or more water-soluble drugs and one or more low-solubility drugs dissolved or dispersed in either a hydrophilic solvent or a fat-soluble solvent, wherein:
(1) the one or more water-soluble drugs are dissolved in a hydrophilic solvent, the one or more low-solubility drugs are lipophilic, low-solubility drugs, and the one or more lipophilic, low-solubility drugs are dispersed in a hydrophilic solvent in a Solid-in-Water (S/W) form comprising the drug-surfactant composite (Solid) dispersed in an aqueous phase (Water); or
(2) the one or more water-soluble drugs are dissolved in a fat-soluble solvent in a Solid-in-Oil (S/O) form comprising the drug-surfactant composite (Solid) dispersed in an oil phase (Oil), and the one or more low-solubility drugs are lipophilic, low-solubility drugs dissolved in a fat-soluble solvent; or
(3) the one or more water-soluble drugs are dispersed in a hydrophilic solvent in a Solid-in-Oil-in-Water (S/O/W) form comprising a Solid-in-Oil (S/O) preparation, further dispersed in an aqueous phase, comprising the drug-surfactant composite (Solid) dispersed in an oil phase (Oil), the one or more low-solubility drugs are lipophilic, low-solubility drugs, and the one or more lipophilic, low-solubility drugs are dispersed in a hydrophilic solvent in a Solid-in-Water (S/W) form comprising the drug-surfactant composite (Solid) dissolved or dispersed in an aqueous phase (Water); or
(4) the one or more water-soluble drugs are dispersed in a fat-soluble solvent in a Solid-in-Oil (S/O) form comprising the drug-surfactant composite (Solid) dispersed in an oil phase (Oil), and the one or more low-solubility drugs are hydrophilic, low-solubility drugs dispersed in a fat-soluble solvent in a Solid-in-Oil (S/O) form comprising the drug-surfactant composite (Solid) dispersed in an oil phase (Oil); or
(5) the one or more water-soluble drugs are dissolved in a hydrophilic solvent, and the one or more hydrophilic, low-solubility drugs are dispersed in a hydrophilic solvent in a Solid-in-Oil-in-Water (S/O/W) form comprising a Solid-in-Oil (S/O) preparation of the drug-surfactant composite (Solid) dispersed in an oil phase (Oil) further dispersed in an aqueous phase; or
(6) the one or more water-soluble drugs are dispersed in aqueous gel, and the one or more low-solubility drugs are hydrophilic, low-solubility drugs dispersed in aqueous gel in a Solid-in-Water (S/W) form comprising the drug-surfactant composite (Solid) dispersed in an aqueous phase (Water);
wherein, in each of cases (1) to (6), the drug-surfactant composite (Solid) refers to a composite that can be prepared by the following steps:
(I) a step of dissolving and/or dispersing drugs in an aqueous solution or organic solvent to obtain a solution and/or dispersion;
(II) a step of liquefying and dissolving a lipophilic or hydrophilic surfactant via heating or other means or dissolving and/or dispersing a lipophilic or hydrophilic surfactant in an organic solvent to obtain a solution and/or dispersion;
(III) a step of mixing the solution and/or dispersion obtained in step (I) with the solution and/or dispersion obtained in step (II) to obtain an emulsion or dispersion; and
(IV) a step of removing a solvent or moisture from the emulsion or dispersion obtained in step (III),
in which step (I) and step (II) may be carried out in any order or simultaneously.

2. The pharmaceutical preparation according to claim 1, wherein the one or more drugs are:
(1) pharmaceutical products selected from among substances having pharmacological, therapeutic, diagnostic, and preventive activity; or
(2) nutritional factors selected from among amino acids, coenzymes including vitamins, minerals, lipids, and saccharides; or
(3) pharmaceutical additives selected from among stabilizers, antioxidants, colorants, antiseptics, preservatives, soothing agents, buffers, amino acids, coenzymes including vitamins, minerals, lipids, and saccharides.

3. A method for producing a drug composition comprising:
(1) dispersing one or more lipophilic, low-solubility drugs and one or more S/O (Solid-in-Oil) water-soluble drugs in a fat-soluble solvent; or
(2) dispersing one or more water-soluble drugs and one or more S/W (Solid-in-Water) lipophilic, low-solubility drugs homogeneously in a hydrophilic solvent; or
(3) dispersing one or more S/O/W (Solid-in-Oil-in-Water) water-soluble drugs and one or more S/W (Solid-in-Water) lipophilic, low-solubility drugs homogeneously in a hydrophilic solvent; or
(4) dispersing one or more S/O (Solid-in-Oil) water-soluble drugs and one or more S/O (Solid-in-Oil) hydrophilic, low-solubility drugs homogeneously in a fat-soluble solvent; or
(5) dispersing one or more water-soluble drugs and one or more S/O/W (Solid-in-Oil-in-Water) hydrophilic, low-solubility drugs homogeneously in a hydrophilic solvent; or
(6) dispersing one or more water-soluble drugs and one or more S/W (Solid-in-Water) hydrophilic, low-solubility drugs homogeneously in a hydrophilic solvent;
wherein, in each of cases (1) to (6), the drug-surfactant composite (Solid) refers to a composite that can be prepared by the following steps:
(I) a step of dissolving and/or dispersing drugs in an aqueous solution or organic solvent to obtain a solution and/or dispersion;
(II) a step of liquefying and dissolving a lipophilic or hydrophilic surfactant via heating or other means or dissolving and/or dispersing a lipophilic or hydrophilic surfactant in an organic solvent to obtain a solution and/or dispersion;
(III) a step of mixing the solution and/or dispersion obtained in step (I) with the solution and/or dispersion obtained in step (II) to obtain an emulsion or dispersion; and
(IV) a step of removing a solvent or moisture from the emulsion or dispersion obtained in step (III),
in which step (I) and step (II) may be carried out in any order or simultaneously.

4. A pharmaceutical, food, or cosmetic product comprising the pharmaceutical preparation according to claim 1 or claim 2.

5. A pharmaceutical preparation comprising soft or hard capsules filled with the pharmaceutical preparation according to claim 1, part (2).

6. A fat-soluble solvent comprising (1) one or more water-soluble drugs homogeneously dispersed therein in an S/O form, and (2) one or more hydrophilic, low-solubility drugs in an S/O form, or lipophilic, low-solubility drugs suspended or dispersed therein;
wherein the drug-surfactant composite (Solid) refers to a composite that can be prepared by the following steps:
(I) a step of dissolving and/or dispersing drugs in an aqueous solution or organic solvent to obtain a solution and/or dispersion;
(II) a step of liquefying and dissolving a lipophilic or hydrophilic surfactant via heating or other means or dissolving and/or dispersing a lipophilic or hydrophilic surfactant in an organic solvent to obtain a solution and/or dispersion;
(III) a step of mixing the solution and/or dispersion obtained in step (I) with the solution and/or dispersion obtained in step (II) to obtain an emulsion or dispersion; and
(IV) a step of removing a solvent or moisture from the emulsion or dispersion obtained in step (III),
in which step (I) and step (II) may be carried out in any order or simultaneously.

7. A pharmaceutical preparation comprising the fat-soluble solvent according to claim 6 filled in soft or hard capsules.

8. A product obtained by packaging the pharmaceutical preparation filled in soft or hard capsules according to claim 7 in an easy-peel or press-through blister pack.

## Patentansprüche

1. Pharmazeutisches Präparat, umfassend ein oder mehrere wasserlösliche Arzneimittel und ein oder mehrere Arzneimittel mit geringer Löslichkeit, die entweder in einem hydrophilen Lösungsmittel oder einem fettlöslichem Lösungsmittel aufgelöst oder dispergiert sind, wobei:
(1) das eine oder die mehreren wasserlöslichen Arzneimittel in einem hydrophilen Lösungsmittel aufgelöst sind, das eine oder die mehreren Arzneimittel mit geringer Löslichkeit lipophile Arzneimittel mit geringer Löslichkeit sind und das eine oder die mehreren lipophilen Arzneimittel mit geringer Löslichkeit in einem hydrophilen Lösungsmittel in einer Feststoff-in-Wasser-Form (S/W-Form) dispergiert sind, die den Arzneimittel-Tensid-Verbund (Feststoff) in einer wässrigen Phase (Wasser) dispergiert umfasst;
(2) das eine oder die mehreren wasserlöslichen Arzneimittel in einem fettlöslichen Lösungsmittel in einer Feststoff-in-Öl-Form (S/O-Form) aufgelöst sind, die den Arzneimittel-Tensid-Verbund (Feststoff) in einer öligen Phase (Öl) dispergiert umfasst, und das eine oder die mehreren Arzneimittel mit geringer Löslichkeit lipophile Arzneimittel mit geringer Löslichkeit sind, die in einem fettlöslichen Lösungsmittel aufgelöst sind; oder
(3) das eine oder die mehreren wasserlöslichen Arzneimittel in einem hydrophilen Lösungsmittel in einer Feststoff-in-Öl-in-Wasser-Form (S/O/W-Form) dispergiert sind, die ein Feststoff-in-Öl-Präparat (S/O-Präparat) umfasst, das zudem in einer wässrigen Phase dispergiert ist, die den Arzneimittel-Tensid-Verbund (Feststoff) in einer öligen Phase (Öl) dispergiert umfasst, das eine oder die mehreren Arzneimittel mit geringer Lösungsmittel lipophile Arzneimittel mit geringer Löslichkeit sind und das eine oder die mehreren lipophilen Arzneimittel mit geringer Löslichkeit in einem hydrophilen Lösungsmittel in einer Feststoff-in-Wasser-Form (S/W-Form) dispergiert sind, die den Arzneimittel-Tensid-Verbund (Feststoff) in einer wässrigen Phase (Wasser) aufgelöst oder dispergiert umfasst; oder
(4) das eine oder die mehreren wasserlöslichen Arzneimittel in einem fettlöslichen Lösungsmittel in einer Feststoff-in-Öl-Form (S/O-Form) dispergiert sind, die den Arzneimittel-Tensid-Verbund (Feststoff) in einer öligen Phase (Öl) dispergiert umfasst, und das eine oder die mehreren Arzneimittel mit geringer Löslichkeit hydrophile Arzneimittel mit geringer Löslichkeit sind, die in einem fettlöslichen Lösungsmittel in einer Feststoff-in-Öl-Form (S/O-Form) dispergiert sind, die den ArzneimittelTensid-Verbund (Feststoff) in einer öligen Phase (Öl) dispergiert umfasst; oder
(5) das eine oder die mehreren wasserlöslichen Arzneimittel in einem hydrophilen Lösungsmittel aufgelöst sind und das eine oder die mehreren hydrophilen Arzneimittel mit geringer Löslichkeit in einem hydrophilen Lösungsmittel in einer Feststoff-in-Öl-in-Wasser-Form (S/O/W-Form) dispergiert sind, die ein Feststoff-in-Öl-Präparat (S/O-Präparat) des Arzneimittel-Tensid-Verbunds (Feststoff) umfasst, der in einer öligen Phase (Öl) dispergiert und in einer wässrigen Phase weiter dispergiert ist; oder
(6) das eine oder die mehreren wasserlöslichen Arzneimittel in einem wässrigen Gel dispergiert sind und das eine oder die mehreren Arzneimittel mit geringer Löslichkeit hydrophile Arzneimittel mit geringer Löslichkeit sind, die in einem wässrigen Gel in einer Feststoff-in-Wasser-Form (S/W-Form) dispergiert sind, die den Arzneimittel-Tensid-Verbund (Feststoff) in einer wässrigen Phase (Wasser) dispergiert umfasst;
wobei in jedem der Fälle (1) bis (6) der Arzneimittel-Tensid-Verbund (Feststoff) einen Verbund betrifft, der durch die folgenden Schritte hergestellt werden kann:
(I) einen Schritt des Auflösens und/oder Dispergierens von Arzneimitteln in einer wässrigen Lösung oder einem organischen Lösungsmittel, um eine Lösung und/oder Dispersion zu erhalten;
(II) einen Schritt des Verflüssigens und Auflösens eines lipophilen oder hydrophilen Tensids durch Erhitzen oder andere Mittel oder das Auflösen und/oder Dispergieren eines lipophilen oder hydrophilen Tensids in einem organischen Lösungsmittel, um eine Lösung und/oder Dispersion zu erhalten;
(III) einen Schritt des Vermischens der in Schritt (I) erhaltenen Lösung und/oder Dispersion mit der in Schritt (II) erhaltenen Lösung und/oder Dispersion, um eine Emulsion oder Dispersion zu erhalten; und
(IV) einen Schritt des Entfernens eines Lösungsmittels oder Feuchtigkeit aus der in Schritt (III) erhaltenen Emulsion oder Dispersion,
wobei Schritt (I) und Schritt (II) in einer beliebigen Reihenfolge oder gleichzeitig durchgeführt werden können.

2. Pharmazeutisches Präparat nach Anspruch 1, wobei das eine oder die mehreren Arzneimittel Folgendes sind:
(1) pharmazeutische Produkte ausgewählt aus Substanzen mit pharmakologischer, therapeutischer, diagnostischer oder präventiver Wirkung; oder
(2) Nahrungsfaktoren ausgewählt aus Aminosäuren, Coenzymen einschließlich Vitaminen, Mineralien, Fetten und Sacchariden; oder
(3) pharmazeutische Additive ausgewählt aus Stabilisatoren, Antioxidanzien, Farbstoffen, Antiseptika, Konservierungsmitteln, Linderungsmitteln, Puffern, Aminosäuren, Coenzymen einschließlich Vitaminen, Mineralien, Fetten und Zuckern.

3. Verfahren zum Herstellen einer Arzneimittelzusammensetzung, das Folgendes umfasst:
(1) das Dispergieren von einem oder mehreren lipophilen Arzneimitteln mit geringer Löslichkeit und einem oder mehreren wasserlöslichen S/O-Arzneimitteln (Feststoff-in-Öl-Arzneimitteln) in einem fettlöslichen Lösungsmittel; oder
(2) das homogene Dispergieren von einem oder mehreren wasserlöslichen Arzneimitteln und einem oder mehreren lipophilen S/W-Arzneimitteln (Feststoff-in-Wasser-Arzneimitteln) mit geringer Löslichkeit in einem hydrophilen Lösungsmittel; oder
(3) das homogene Dispergieren von einem oder mehreren wasserlöslichen S/O/W-Arzneimitteln (Feststoff-in-Öl-in-Wasser-Arzneimitteln) und einem oder mehreren lipophilen S/W-Arzneimitteln (Feststoff-in-Wasser-Arzneimitteln) mit geringer Löslichkeit in einem hydrophilen Lösungsmittel; oder
(4) das homogene Dispergieren von einem oder mehreren wasserlöslichen S/O-Arzneimitteln (Feststoff-in-Öl-Arzneimitteln) und einem oder mehreren hydrophilen S/O-Arzneimitteln (Feststoff-in-Öl-Arzneimitteln) mit geringer Löslichkeit in einem fettlöslichen Lösungsmittel; oder
(5) das homogene Dispergieren von einem oder mehreren wasserlöslichen Arzneimitteln und einem oder mehreren hydrophilen S/O/W-Arzneimitteln (Feststoff-in-Öl-in-Wasser-Arzneimitteln) mit geringer Löslichkeit in einem hydrophilen Lösungsmittel; oder
(6) das homogene Dispergieren von einem oder mehreren wasserlöslichen Arzneimitteln und einem oder mehreren hydrophilen S/W-Arzneimitteln (Feststoff-in-Wasser-Arzneimitteln) mit geringer Löslichkeit in einem hydrophilen Lösungsmittel;
wobei in jedem der Fälle (1) bis (6) der Arzneimittel-Tensid-Verbund (Feststoff) einen Verbund betrifft, der durch die folgenden Schritte hergestellt werden kann:
(I) einen Schritt des Auflösens und/oder Dispergierens von Arzneimitteln in einer wässrigen Lösung oder einem organischen Lösungsmittel, um eine Lösung und/oder Dispersion zu erhalten;
(II) einen Schritt des Verflüssigens und Auflösens eines lipophilen oder hydrophilen Tensids durch Erhitzen oder andere Mittel oder das Auflösen und/oder Dispergieren eines lipophilen oder hydrophilen Tensids in einem organischen Lösungsmittel, um eine Lösung und/oder Dispersion zu erhalten;
(III) einen Schritt des Vermischens der in Schritt (I) erhaltenen Lösung und/oder Dispersion mit der in Schritt (II) erhaltenen Lösung und/oder Dispersion, um eine Emulsion oder Dispersion zu erhalten; und
(IV) einen Schritt des Entfernens eines Lösungsmittels oder Feuchtigkeit aus der in Schritt (III) erhaltenen Emulsion oder Dispersion,
wobei Schritt (I) und Schritt (II) in einer beliebigen Reihenfolge oder gleichzeitig durchgeführt werden können.

4. Pharmazie-, Nahrungs- oder Kosmetikprodukt, umfassend das pharmazeutische Präparat nach Anspruch 1 oder 2.

5. Pharmazeutisches Präparat, umfassend Weich- oder Hartkapseln, die mit dem pharmazeutischen Präparat nach Anspruch 1, Teil (2) gefüllt sind.

6. Fettlösliches Lösungsmittel, umfassend (1) ein oder mehrere darin homogen dispergierte wasserlösliche Arzneimittel in einer S/O-Form und (2) ein oder mehrere hydrophile Arzneimittel mit geringer Löslichkeit in einer S/O-Form oder darin suspendierte oder dispergierte lipophile Arzneimittel mit geringer Löslichkeit; wobei der Arzneimittel-Tensid-Verbund (Feststoff) einen Verbund betrifft, der durch die folgenden Schritte hergestellt werden kann:
(I) einen Schritt des Auflösens und/oder Dispergierens von Arzneimitteln in einer wässrigen Lösung oder einem organischen Lösungsmittel, um eine Lösung und/oder Dispersion zu erhalten;
(II) einen Schritt des Verflüssigens und Auflösens eines lipophilen oder hydrophilen Tensids durch Erhitzen oder andere Mittel oder das Auflösen und/oder Dispergieren eines lipophilen oder hydrophilen Tensids in einem organischen Lösungsmittel, um eine Lösung und/oder Dispersion zu erhalten;
(III) einen Schritt des Vermischens der in Schritt (I) erhaltenen Lösung und/oder Dispersion mit der in Schritt (II) erhaltenen Lösung und/oder Dispersion, um eine Emulsion oder Dispersion zu erhalten; und
(IV) einen Schritt des Entfernens eines Lösungsmittels oder Feuchtigkeit aus der in Schritt (III) erhaltenen Emulsion oder Dispersion,
wobei Schritt (I) und Schritt (II) in einer beliebigen Reihenfolge oder gleichzeitig durchgeführt werden können.

7. Pharmazeutisches Präparat, umfassend das in Weich- oder Hartkapseln gefüllte fettlösliche Lösungsmittel nach Anspruch 6.

8. Produkt, erhalten durch Verpacken des in Weich- oder Hartkapseln gefüllten pharmazeutischen Präparats nach Anspruch 7 in eine leicht abzuziehende oder herauszudrückende Blisterverpackung.

## Revendications

1. Préparation pharmaceutique comprenant un ou plusieurs médicaments solubles dans l'eau et un ou plusieurs médicaments présentant une faible solubilité dissous ou dispersés soit dans un solvant hydrophile soit dans un solvant liposoluble, dans laquelle :
(1) le ou les médicaments solubles dans l'eau sont dissous dans un solvant hydrophile, le ou les médicaments présentant une faible solubilité sont des médicaments lipophiles présentant une faible solubilité, et le ou les médicaments lipophiles présentant une faible solubilité sont dispersés dans un solvant hydrophile sous la forme d'un solide dans l'eau (S/E) comprenant le composite médicament-tensioactif (solide) dispersé dans une phase aqueuse (eau) ; ou
(2) le ou les médicaments solubles dans l'eau sont dissous dans un solvant liposoluble sous la forme d'un solide dans l'huile (S/H) comprenant le composite médicament-tensioactif (solide) dispersé dans une phase huileuse (huile), et le ou les médicaments présentant une faible solubilité sont des médicaments lipophiles présentant une faible solubilité dissous dans un solvant liposoluble ; ou
(3) le ou les médicaments solubles dans l'eau sont dispersés dans un solvant hydrophile sous la forme d'un solide dans l'huile dans l'eau (S/H/E) comprenant une préparation sous la forme d'un solide dans l'huile (S/H), elle-même dispersée dans une phase aqueuse, comprenant le composite médicament-tensioactif (solide) dispersé dans une phase huileuse (huile), le ou les médicaments présentant une faible solubilité sont des médicaments lipophiles présentant une faible solubilité, et le ou les médicaments lipophiles présentant une faible solubilité sont dispersés dans un solvant hydrophile sous la forme d'un solide dans l'eau (S/E) comprenant le composite médicament-tensioactif (solide) dissous ou dispersé dans une phase aqueuse (eau) ; ou
(4) le ou les médicaments solubles dans l'eau sont dispersés dans un solvant liposoluble sous la forme d'un solide dans l'huile (S/H) comprenant le composite médicament-tensioactif (solide) dispersé dans une phase huileuse (huile), et le ou les médicaments présentant une faible solubilité sont des médicaments hydrophiles présentant une faible solubilité dispersés dans un solvant liposoluble sous la forme d'un solide dans l'huile (S/H) comprenant le composite médicament-tensioactif (solide) dispersé dans une phase huileuse (huile) ; ou
(5) le ou les médicaments solubles dans l'eau sont dissous dans un solvant hydrophile, et le ou les médicaments hydrophiles présentant une faible solubilité sont dispersés dans un solvant hydrophile sous la forme d'un solide dans l'huile dans l'eau (S/H/E) comprenant une préparation sous la forme d'un solide dans l'huile (S/H) du composite médicament-tensioactif (solide) dispersé dans une phase huileuse (huile) elle-même dispersée dans une phase aqueuse ; ou
(6) le ou les médicaments solubles dans l'eau sont dispersés dans un gel aqueux, et le ou les médicaments présentant une faible solubilité sont des médicaments hydrophiles présentant une faible solubilité dispersés dans un gel aqueux sous la forme d'un solide dans l'eau (S/E) comprenant le composite médicament-tensioactif (solide) dispersé dans une phase aqueuse (eau) ;
dans laquelle, dans chacun des cas (1) à (6), le composite médicament-tensioactif (solide) désigne un composite qui peut être préparé par les étapes suivantes :
(I) une étape de dissolution et/ou dispersion des médicaments dans une solution aqueuse ou un solvant organique pour obtenir une solution et/ou une dispersion ;
(II) une étape de liquéfaction et dissolution d'un tensioactif lipophile ou hydrophile par chauffage ou d'autres moyens ou de dissolution et/ou dispersion d'un tensioactif lipophile ou hydrophile dans un solvant organique pour obtenir une solution et/ou une dispersion ;
(III) une étape de mélange de la solution et/ou de la dispersion obtenue à l'étape (I) avec la solution et/ou la dispersion obtenue à l'étape (II) pour obtenir une émulsion ou une dispersion ; et
(IV) une étape d'élimination d'un solvant ou de l'humidité de l'émulsion ou de la dispersion obtenue à l'étape (III),
dans laquelle l'étape (I) et l'étape (II) peuvent être réalisées dans un ordre quelconque ou simultanément.

2. Préparation pharmaceutique selon la revendication 1, dans laquelle le ou les médicaments sont :
(1) des produits pharmaceutiques choisis parmi les substances ayant une activité pharmacologique, thérapeutique, diagnostique et préventive ; ou
(2) des facteurs nutritionnels choisis parmi les acides aminés, les coenzymes incluant les vitamines, les minéraux, les lipides et les saccharides ; ou
(3) des additifs pharmaceutiques choisis parmi les stabilisants, les antioxydants, les colorants, les antiseptiques, les conservateurs, les agents apaisants, les tampons, les acides aminés, les coenzymes incluant les vitamines, les minéraux, les lipides et les saccharides.

3. Procédé de production d'une composition de médicament comprenant :
(1) la dispersion d'un ou plusieurs médicaments lipophiles présentant une faible solubilité et d'un ou plusieurs médicaments solubles dans l'eau sous forme S/H (solide dans l'huile) dans un solvant liposoluble ; ou
(2) la dispersion d'un ou plusieurs médicaments solubles dans l'eau et d'un ou plusieurs médicaments lipophiles présentant une faible solubilité sous forme S/E (solide dans l'eau) de manière homogène dans un solvant hydrophile ; ou
(3) la dispersion d'un ou plusieurs médicaments solubles dans l'eau sous forme S/H/E (solide dans l'huile dans l'eau) et d'un ou plusieurs médicaments lipophiles présentant une faible solubilité sous forme S/E (solide dans l'eau) de manière homogène dans un solvant hydrophile ; ou
(4) la dispersion d'un ou plusieurs médicaments solubles dans l'eau sous forme S/H (solide dans l'huile) et d'un ou plusieurs médicaments hydrophiles présentant une faible solubilité sous forme S/H (solide dans l'huile) de manière homogène dans un solvant liposoluble ; ou
(5) la dispersion d'un ou plusieurs médicaments solubles dans l'eau et d'un ou plusieurs médicaments hydrophiles présentant une faible solubilité sous forme S/H/E (solide dans l'huile dans l'eau) de manière homogène dans un solvant hydrophile ; ou
(6) la dispersion d'un ou plusieurs médicaments solubles dans l'eau et d'un ou plusieurs médicaments hydrophiles présentant une faible solubilité sous forme S/E (solide dans l'eau) de manière homogène dans un solvant hydrophile ;
dans lequel, dans chacun des cas (1) à (6), le composite médicament-tensioactif (solide) désigne un composite qui peut être préparé par les étapes suivantes :
(I) une étape de dissolution et/ou dispersion des médicaments dans une solution aqueuse ou un solvant organique pour obtenir une solution et/ou une dispersion ;
(II) une étape de liquéfaction et dissolution d'un tensioactif lipophile ou hydrophile par chauffage ou d'autres moyens ou de dissolution et/ou dispersion d'un tensioactif lipophile ou hydrophile dans un solvant organique pour obtenir une solution et/ou une dispersion ;
(III) une étape de mélange de la solution et/ou de la dispersion obtenue à l'étape (I) avec la solution et/ou la dispersion obtenue à l'étape (II) pour obtenir une émulsion ou une dispersion ; et
(IV) une étape d'élimination d'un solvant ou de l'humidité de l'émulsion ou de la dispersion obtenue à l'étape (III),
dans lequel l'étape (I) et l'étape (II) peuvent être réalisées dans un ordre quelconque ou simultanément.

4. Produit pharmaceutique, alimentaire ou cosmétique comprenant la préparation pharmaceutique selon la revendication 1 ou la revendication 2.

5. Préparation pharmaceutique comprenant des capsules molles ou dures remplies de la préparation pharmaceutique selon la revendication 1, partie (2).

6. Solvant liposoluble comprenant (1) un ou plusieurs médicaments solubles dans l'eau y étant dispersés de manière homogène sous une forme S/H, et (2) un ou plusieurs médicaments hydrophiles présentant une faible solubilité sous une forme S/H, ou médicaments lipophiles présentant une faible solubilité y étant en suspension ou dispersés ;
dans lequel le composite médicament-tensioactif (solide) désigne un composite qui peut être préparé par les étapes suivantes :
(I) une étape de dissolution et/ou dispersion des médicaments dans une solution aqueuse ou un solvant organique pour obtenir une solution et/ou une dispersion ;
(II) une étape de liquéfaction et dissolution d'un tensioactif lipophile ou hydrophile par chauffage ou d'autres moyens ou de dissolution et/ou dispersion d'un tensioactif lipophile ou hydrophile dans un solvant organique pour obtenir une solution et/ou une dispersion ;
(III) une étape de mélange de la solution et/ou de la dispersion obtenue à l'étape (I) avec la solution et/ou la dispersion obtenue à l'étape (II) pour obtenir une émulsion ou une dispersion ; et
(IV) une étape d'élimination d'un solvant ou de l'humidité de l'émulsion ou de la dispersion obtenue à l'étape (III),
dans laquelle l'étape (I) et l'étape (II) peuvent être réalisées dans un ordre quelconque ou simultanément.

7. Préparation pharmaceutique comprenant le solvant liposoluble selon la revendication 6 chargé dans des capsules molles ou dures.

8. Produit obtenu en conditionnant la préparation pharmaceutique chargée dans des capsules molles ou dures selon la revendication 7 dans un emballage-coque facile à décoller ou à percer.
